# FASCICULE DE BREVET EUROPEEN

(11) **EP 3 464 396 B1**
(45) Date de publication et mention de la délivrance du brevet: **29.06.2022**
(21) Numéro de dépôt: 17735184.8
(22) Date de dépôt: 02.06.2017
(51) Int. Cl.: C08F 220/06, C08F 220/28, C08F 265/06

(54) **COPOLYMÈRE ÉPAISSISSANT ET SUSPENSIF**
COPOLYMER MIT VERDICKUNGS- UND SUSPENSIONSEIGENSCHAFTEN
COPOLYMER HAVING THICKENING AND SUSPENSION PROPERTIES

(30) Priorité: 03.06.2016 FR 1655055
(43) Date de publication de la demande: 10.04.2019
(73) Titulaire: Coatex, 69730 Genay (FR)
(72) Inventeur: CAVALIÉ, Hervé, 60300 Senlis (FR); CHAMPAGNE, Clémentine, 69300 Caluire-Et-Cuire (FR); MAGNY, Benoît, 69270 Cailloux Sur Fontaine (FR); SUAU, Jean-Marc, 69480 Lucenay (FR); VERGÉ, Christophe, 60580 Coye La Foret (FR)
(74) Mandataire: Balmefrezol, Ludovic Francis Pierre
(86) Numéro de dépôt international: PCT/FR2017/051392
(87) Numéro de publication internationale: WO 2017/207944

(56) Documents cités:
- EP-A1- 2 620 466
- FR-A- 1 329 008
- FR-A1- 3 000 085
- US-A1- 2016 083 532

## Description

L'invention concerne le domaine de la préparation de compositions aqueuses comprenant des agents modificateurs de rhéologie, notamment la préparation de compositions aqueuses cosmétiques ou détergentes possédant des propriétés d'épaississement et de clarté améliorées ainsi que de bonnes propriétés suspensivantes.

En particulier, l'invention concerne un agent modificateur de rhéologie qui est un copolymère obtenu par polymérisation d'au moins un monomère réticulant avec au moins un monomère anionique comprenant au moins une insaturation éthylénique polymérisable et au moins un monomère non-ionique hydrophobe comprenant au moins une insaturation éthylénique polymérisable.

On connaît des agents modificateurs de rhéologie, également appelés agents épaississants ou agents de viscosité. Généralement, ils sont présents dans les compositions nettoyantes, par exemple dans des compositions de soin ou d'hygiène de la personne, notamment des compositions cosmétiques, ou dans des compositions d'entretien, notamment dans des produits détergents. De manière habituelle, ces compositions sont riches en composés tensioactifs.

Ces agents influencent les propriétés rhéologiques de la formulation, en particulier la viscosité, ainsi que les propriétés optiques ou esthétiques, telle que la clarté. Ces agents influencent également la capacité à suspendre et à stabiliser des particules au sein de la formulation.

Parmi les agents modificateurs de rhéologie couramment utilisés dans les formulations aqueuses, on peut citer les copolymères solubles ou gonflables en milieu alcalin (polymères ASE pour Alkali-Soluble Emulsions ou Alkali-Swellable Emulsions). On peut également citer les copolymères solubles ou gonflables en milieu alcalin et modifiés hydrophobiquement (polymères HASE pour Hydrophobically modified Alkali-Soluble Emulsions ou Hydrophobically modified Alkali-Swellable Emulsions).

On connaît également des compositions aqueuses comprenant des copolymères ASE ou des copolymères HASE comme agents modificateurs de rhéologie.

Pour ces compositions aqueuses, on cherche notamment à améliorer leurs propriétés ou leurs performances pour une large gamme de pH. En particulier, on cherche à obtenir des compositions aqueuses possédant une clarté élevée, de bonnes propriétés en termes d'effet épaississant ainsi que de bonnes propriétés suspensivantes.

Le contrôle de la viscosité et l'obtention de compositions aqueuses sous la forme d'une phase continue et limpide sont particulièrement recherchés, en particulier pour une large gamme de pH.

Ainsi, les propriétés et les performances des compositions aqueuses doivent pouvoir être mises en œuvre tant à des valeurs de pH acide qu'à des valeurs de pH neutre ou basique.

Une composition aqueuse possède de bonnes propriétés suspensivantes ou un bon pouvoir suspensif lorsqu'elle est capable de maintenir en suspension des particules dans sa phase continue. Cette capacité doit pouvoir durer dans le temps afin d'obtenir des compositions aqueuses stables, par exemple lors de leur stockage.

Généralement, les propriétés suspensivantes sont évaluées par application d'un test applicatif de suspension qui permet de déterminer la valeur du module d'élasticité G', la valeur de Tan (δ) et la valeur de la résistance élastique de la composition aqueuse comprenant un agent modificateur de rhéologie.

De manière générale, les particules à suspendre dans la phase continue de la composition aqueuse sont des corps solides, pleins ou creux. Ces particules à suspendre peuvent également être des entités liquides non miscibles avec la phase continue de la composition aqueuse ou encore des corps encapsulés ou des corps gazeux qui peuvent être caractérisés par des formes, des textures, des structures, des compositions, des couleurs et des propriétés finales différentes.

A titre indicatif, on peut citer les particules exfoliantes, par exemple les particules de polyéthylène, les coquilles de fruits pilées, les pierres ponces. On peut également citer les particules nourrissantes, par exemple les sphères de collagène, ainsi que les particules nacrantes, par exemple le mica titane, les glycols distéarates, ou encore les particules esthétiques, par exemple les bulles d'air, les paillettes, les pigments éventuellement colorés.

Habituellement, les particules à suspendre peuvent être de taille assez variable. Par exemple, les bulles d'air peuvent avoir une taille de 1, 2 ou 3 mm.

La clarté ou la limpidité des compositions aqueuses peuvent être évaluées par mesure de leur transmittance, généralement exprimée en pourcentage. Une composition est considérée claire ou limpide si elle présente une transmittance, pour une longueur d'onde de 500 nm, d'au moins 60 %, de préférence d'au moins 70 % et plus préférentiellement encore d'au moins 80 %.

Le document FR 3000085 divulgue la préparation d'une composition aqueuse pour gel douche comprenant des particules en suspension dans une phase continue limpide. Le composé réticulant utilisé est le diméthacrylate d'éthylène glycol (EDMA) ou bien le triméthacrylate de triméthylolpropane (TMP-TMA).

Le document FR 1363955 décrit la préparation d'esters de monoacides aliphatiques carboxyliques α,β-éthyléniques et d'alcool homopérillique.

Le document US 2004 0213892 décrit un polymère revêtu d'un tensio-actif azoté non-ionique. Le document EP 2780382 divulgue une émulsion latex polymérique utilisable comme agent épaississant.

Le document EP 2620466 concerne une méthode de traitement thermique de particules de polymère hydroabsorbant. Le document US 2016 0083532 décrit la préparation de particules de polymère hydroabsorbant.

Les agents modificateurs de rhéologie de l'état de la technique et les compositions aqueuses de l'état de la technique les comprenant ne donnent pas toujours satisfaction et conduisent à des problèmes liés à ces nombreuses propriétés recherchées.

Il existe donc un besoin pour l'homme du métier de disposer d'agents modificateurs de rhéologie possédant des propriétés améliorées, en particulier des propriétés présentes au sein de compositions aqueuses.

L'invention permet d'apporter une solution à tout ou partie des problèmes rencontrés avec les agents modificateurs de rhéologie de l'état de la technique.

Ainsi, l'invention fournit un copolymère (P1) obtenu par réaction de polymérisation :
(a) d'au moins un monomère anionique comprenant au moins une insaturation éthylénique polymérisable ; et
(b) d'au moins un monomère non-ionique hydrophobe comprenant au moins une insaturation éthylénique polymérisable et choisi parmi les acrylates de C₁-C₈-alkyle, les méthacrylates de C₁-C₈-alkyle, les maléates de C₁-C₈-alkyle, les itaconates de C₁-C₈-alkyle, les crotonates de C₁-C₈-alkyle, les cinnamates de C₁-C₈-alkyle et leurs mélanges ; et
(c) d'au moins un monomère de formule (I) : 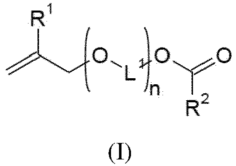 dans laquelle :
   - R¹ représente indépendamment H ou CH3 ;
   - R² représente indépendamment -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH ;
   - L¹ représente indépendamment un groupement éthylène, propylène ou butylène ;
   - n représente indépendamment 0 ou un nombre entier ou décimal allant de 1 à 30.

Selon l'invention, lors de la réaction de polymérisation, les monomères peuvent être introduits séparément ou bien sous la forme d'un ou plusieurs mélanges de ces monomères.

De manière préférée, les monomères sont introduits sous la forme d'un mélange.

De manière préférée selon l'invention, le monomère anionique (a) est un monomère anionique comprenant une fonction vinylique polymérisable et au moins une fonction acide carboxylique. De manière plus préférée, il s'agit d'un monomère choisi parmi l'acide acrylique, l'acide méthacrylique, l'acide maléique, l'acide itaconique, l'acide crotonique, un sel d'acide acrylique, un sel d'acide méthacrylique, un sel d'acide maléique, un sel d'acide itaconique, un sel d'acide crotonique, un sel d'acide cinnamique, et leurs mélanges.

De manière plus préférée selon l'invention, le monomère anionique (a) est choisi parmi l'acide acrylique, l'acide méthacrylique, un sel d'acide acrylique, un sel d'acide méthacrylique et leurs mélanges. De manière encore plus préférée selon l'invention, le monomère anionique (a) est choisi parmi l'acide acrylique, l'acide méthacrylique et leurs mélanges. Le monomère (a) plus particulièrement préféré est l'acide méthacrylique.

De manière également préférée selon l'invention, le monomère anionique (a) est mis en œuvre en une quantité d'au moins 20 % molaire, de préférence de 25 à 60 % molaire, en particulier de 30 à 55 % molaire, par rapport à la quantité molaire totale de monomères.

Le monomère non-ionique hydrophobe (b) est choisi parmi les acrylates de C₁-C₈-alkyle, les méthacrylates de C₁-C₈-alkyle, les maléates de C₁-C₈-alkyle, les itaconates de C₁-C₈-alkyle, les crotonates de C₁-C₈-alkyle, les cinnamates de C₁-C₈-alkyle et leurs mélanges, de préférence l'acrylate de méthyle, l'acrylate d'éthyle, l'acrylate de butyle, l'acrylate de 2-éthyle-hexyle, le méthacrylate de méthyle, le méthacrylate d'éthyle, le méthacrylate de butyle et leurs mélanges. De manière particulièrement préférée, le monomère non-ionique hydrophobe (b) est choisi parmi l'acrylate de méthyle, l'acrylate d'éthyle, l'acrylate de butyle, le méthacrylate de méthyle, le méthacrylate d'éthyle et leurs mélanges. Le monomère non-ionique hydrophobe (b) plus particulièrement préféré est l'acrylate d'éthyle.

De manière également préférée selon l'invention, le monomère non-ionique hydrophobe (b) est mis en œuvre en une quantité de 30 à 80 % molaire, de préférence de 35 à 75 % molaire, plus préférentiellement de 45 à 70 % molaire, par rapport à la quantité molaire totale de monomères.

De manière particulièrement préférée, le copolymère (P1) peut être préparé à partir de monomère anionique (a) choisi parmi l'acide acrylique, l'acide méthacrylique et leurs mélanges, préférentiellement l'acide méthacrylique, et de monomère non-ionique hydrophobe (b) choisi parmi l'acrylate de méthyle, l'acrylate d'éthyle, l'acrylate de butyle, le méthacrylate de méthyle, le méthacrylate d'éthyle et leurs mélanges, préférentiellement l'acrylate d'éthyle.

Outre les monomères (a) et (b), la préparation du copolymère (P1) selon l'invention met en œuvre au moins un monomère de formule (I). Ce monomère de formule (I) comprend au moins deux insaturations éthyléniques polymérisables. Le monomère de formule (I) est avantageusement un monomère réticulant, plus avantageusement : un monomère qui n'est pas un monomère associatif. De manière particulièrement essentielle, le monomère de formule (I) est un monomère réticulant dont les deux insaturations éthyléniques polymérisables ont des propriétés différentes qui confèrent des propriétés réticulantes spécifiques au monomère de formule (I).

De manière préférée, la préparation du copolymère (P1) selon l'invention met en œuvre un ou deux monomères de formule (I), en particulier un monomère de formule (I).

Pour le monomère (c), L¹ représente indépendamment un groupement éthylène, propylène ou butylène, à savoir respectivement un groupement CH2-CH2, CH(CH3)-CH2 et CH(CH₂CH₃)-CH₂.

De manière préférée selon l'invention, le monomère (c) est un composé de formule (I) dans laquelle n représente un nombre entier ou décimal allant de 1 à 18, de 1 à 15 ou de 2 à 16 ou encore de 2 à 12.

De manière particulièrement préférée selon l'invention, le monomère (c) est un composé (c1) de formule (I) dans laquelle R¹ représente H; R² représente -C(H)=CH₂ ; L¹ représente CH₂-CH₂ et n représente 10 (numéro CAS 99742-80-0). De manière également particulièrement préférée selon l'invention, le monomère (c) est un composé (c2) de formule (I) dans laquelle R¹ représente H; R² représente -C(CH₃)=CH_{2;} L¹ représente CH₂-CH₂ et n représente 3,5 (numéro CAS 121826-50-4).

De manière également particulièrement préférée selon l'invention, le monomère (c) est un composé (c3) de formule (I) dans laquelle R¹ représente H ; L¹ représente CH₂-CH_{2;} R² représente -C(CH₃)=CH₂ et n représente 10 (numéro CAS 121826-50-4). Ces composés de formule (I) sont connus en tant que tels et peuvent être préparés selon les méthodes décrites dans l'état de la technique ou selon des méthodes pouvant être adaptées des méthodes décrites dans l'état de la technique.

De manière également préférée selon l'invention, le monomère (c) est mis en œuvre en une quantité de moins de 5 % molaire, de préférence de 0,01 à 5 % molaire et plus préférentiellement de 0,02 à 4 % molaire ou de 0,02 à 2 % molaire ou encore de 0,04 à 0,5 % molaire ou de 0,04 à 0,25 % molaire, de monomère (c) par rapport à la quantité molaire totale de monomères.

Le monomère (c) est généralement connu en tant que tel ou bien il peut être préparé à partir des méthodes de préparation décrites dans le document US 2006-0052564.

Selon l'invention, le copolymère (P1) est préparé par une réaction de polymérisation comprenant la mise en œuvre de monomères (a) et (b) et de monomère (c) de formule (I). Le copolymère (P1) peut donc être préparé à partir de ces seuls monomères (a), (b) et (c) de formule (I).

Le copolymère (P1) peut également être préparé à partir de ces trois types de monomères associés à d'autres monomères. Ainsi, outre les monomères (a) et (b) et le monomère (c) de formule (I), la réaction de polymérisation pour préparer le copolymère (P1) peut mettre en œuvre un ou plusieurs autres monomères.

De manière avantageuse, le copolymère (P1) peut être préparé par une réaction de polymérisation mettant également en œuvre au moins un monomère non-ionique (d), différent du monomère (b), comprenant une fonction vinylique polymérisable et une chaîne hydrocarbonée comprenant au moins 10 atomes de carbone. Selon l'invention, le monomère non-ionique (d) est, de préférence, choisi parmi :
- un monomère (d1) comprenant une fonction vinylique polymérisable et une chaîne hydrocarbonée en C₁₂-C₃₆;
- un monomère (d2) comprenant une fonction vinylique polymérisable, une chaîne hydrocarbonée en C₁₂-C₃₆ et de 1 à 150, de préférence de 15 à 50 et plus préférentiellement de 20 à 30, groupements alkylène-oxy.

Pour le monomère non-ionique (d2), les groupements oxy-alkylènes préférés sont les groupements éthoxy (OE), propoxy (PO) et butoxy (BO), en particulier le groupement éthoxy (OE). Un monomère non-ionique (d2) préféré est un composé de formule (II) :

T¹-(CH₂CH₂O)ₘ-(CH₂CH(CH₃)O)ₚ-(CH₂CH(CH₂CH₃)O)_{q}-T² (II)

dans laquelle :
- T¹ représente indépendamment une fonction vinylique polymérisable ;
- T² représente indépendamment :
   ∘ une chaîne hydrocarbonée comprenant au moins 10 atomes de carbone, de préférence une chaîne hydrocarbonée en C₁₂-C₃₆ ; ou
   ∘ une chaîne hydrocarbonée comprenant au moins 10 atomes de carbone, de préférence une chaîne hydrocarbonée en C₁₂-C₃₆, et au moins un hétéroatome choisi parmi O, S, N et P ; ou
- m, p et q, identiques ou différents, représentent indépendamment un nombre entier ou décimal allant de 0 à 150, la somme de m, p et q étant non-nulle.

De manière préférée, T¹ représente une fonction vinylique polymérisable choisie parmi un groupement vinyl, un groupement méthylvinyl, un groupement acrylate, un groupement méthacrylate, un groupement allyl, un groupement méthallyl.

Comme composé (d2) particulier, on peut citer le composé (d2-1) de formule (II) dans laquelle T¹ représente un groupement -OC(O)C(CH₃)=CH₂, T² représente une chaîne hydrocarbonée ramifiée comportant 16 atomes de carbone (2-hexyldécanyl), m représente 25 et p et q représentent 0.

De manière également avantageuse, la réaction de polymérisation peut mettre en œuvre de 0,01 à 10 % molaire de monomère (d) par rapport à la quantité molaire totale de monomères. De manière préférée, la réaction de polymérisation peut mettre en œuvre de 0,02 à 5 % molaire ou de 0,02 à 2 % molaire, de monomère (d) par rapport à la quantité molaire totale de monomères.

De manière avantageuse, le copolymère (P1) peut être préparé par une réaction de polymérisation mettant également en œuvre au moins un monomère ionique ou non-ionique (e), différent du monomère (b). De préférence selon l'invention, le monomère ionique ou non-ionique (e) est choisi parmi :
- l'acide 2-acrylamido-2-méthylpropane sulfonique ou un de ses sels ;
- les télomères, de préférence les dimères, les trimères ou les tétramères, insaturés de l'acide acrylique ;
- les monomères de formule (III) : 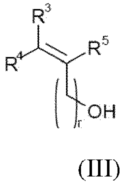 dans laquelle :
   ▪ R³, R⁴ et R⁵, identiques ou différents, représentent indépendamment H ou CH3 ;
   ▪ r représente indépendamment 1, 2 ou 3 ;
- les monomères de formule (IV) : 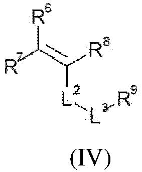 dans laquelle :
   ▪ R⁶, R⁷ et R⁸, identiques ou différents, représentent indépendamment H ou CH3 ;
   ▪ R⁹ représente H ou CH3 ;
   ▪ L² représente indépendamment une liaison directe ou un groupement choisi parmi O, C(=O)O, CH2CH2 et CH2 ;
   ▪ L³ représente indépendamment une liaison directe ou de 1 à 150, de préférence de 15 à 50 et plus préférentiellement de 25 à 30, groupements alkylène-oxy.

Pour le monomère ionique ou non-ionique (e), les groupements oxy-alkylènes préférés sont les groupements éthoxy (OE), propoxy (PO) et butoxy (BO), en particulier le groupement éthoxy (OE).

Selon l'invention, un groupement éthoxy (OE) est un résidu -CH₂-CH₂-O, un groupement propoxy (PO) est un groupement éthoxy substitué par un radical méthyle sur l'un des atomes de carbone en remplacement d'un atome d'hydrogène et un groupement butoxy (BO) est un groupement éthoxy substitué par un radical éthyle sur l'un des atomes de carbone en remplacement d'un atome d'hydrogène.

Selon l'invention, parmi les monomères (e) de formule (IV) préférés, on connaît :
- HEMA ou hydroxyéthyle méthacrylate qui est un composé de formule (IV) dans laquelle R⁶, R⁷ et R⁹ représentent H, R⁸ représente CH3, L² représente C(O)O et L³ représente un groupement éthylène-oxy ; et
- HPMA ou hydroxypropyle méthacrylate qui est un composé de formule (IV) dans laquelle R⁶, R⁷ et R⁹ représentent H, R⁸ représente CH3, L² représente C(O)O et L³ représente un groupement propylène-oxy ;
- HPA ou hydroxypropyle acrylate qui est un composé de formule (IV) dans laquelle R⁶, R⁷, R⁸ et R⁹ représentent H, L² représente C(O)O et L³ représente un groupement propylène-oxy.

De manière également avantageuse, la réaction de polymérisation peut mettre en œuvre de 0,01 à 25 % molaire de monomère (e) par rapport à la quantité molaire totale de monomères. De manière préférée, la réaction de polymérisation peut mettre en œuvre de 0,02 à 15 % molaire ou de 0,02 à 10 % molaire, de monomère (e) par rapport à la quantité molaire totale de monomères.

De manière avantageuse, le copolymère (P1) peut être préparé par une réaction de polymérisation mettant également en œuvre au moins un monomère (f). Le monomère (f) est avantageusement un monomère réticulant, hydrophile, hydrophobe ou amphiphile et il est généralement un composé comprenant plusieurs insaturations éthyléniques. Il est distinct du monomère de formule (I) selon l'invention. Le monomère (f) peut être un composé choisi parmi :
- un composé de formule de formule (VI) : 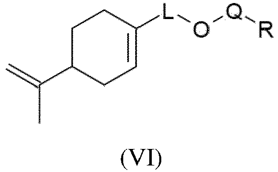 dans laquelle :
   - L représente CH2, CH2 monoalkoxylé ou CH2 polyalkoxylé ;
   - Q représente une liaison directe ou C(O) ;
   - R représente -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ ou Q³OQ⁴OC(O)C(H)=CH₂;
   - Q³ représente un résidu divalent d'un composé diisocyanate dissymétrique, de préférence choisi parmi tolyl-1,3-diisocyanate (TDI) et isophorone-diisocyanate (IPDI) ;
   - Q⁴ représente CH2, CH2-CH2, CH2 monoalkoxylé, CH2-CH2 monoalkoxylé, CH2 polyalkoxylé ou CH2-CH2 polyalkoxylé ;
- un composé de formule (VI-A) : 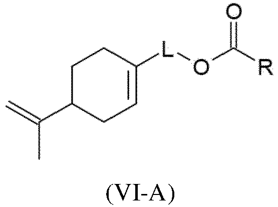 dans laquelle :
   - L représente CH2, CH2 monoalkoxylé ou CH2 polyalkoxylé ;
   - R représente -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ ou Q³OQ⁴OC(O)C(H)=CH₂ ;
   - Q³ représente un résidu divalent d'un composé diisocyanate dissymétrique, de préférence choisi parmi tolyl-1,3-diisocyanate (TDI) et isophorone-diisocyanate (IPDI) ;
   - Q⁴ représente CH2, CH2-CH2, CH2 monoalkoxylé, CH2-CH2 monoalkoxylé, CH2 polyalkoxylé ou CH2-CH2 polyalkoxylé ;
- un composé de formule (VI-B) : 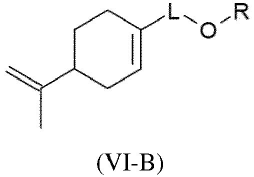 dans laquelle :
   - L représente CH2, CH2 monoalkoxylé ou CH2 polyalkoxylé ;
   - R représente -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ ou Q³OQ⁴OC(O)C(H)=CH₂ ;
   - Q³ représente un résidu divalent d'un composé diisocyanate dissymétrique, de préférence choisi parmi tolyl-1,3-diisocyanate (TDI) et isophorone-diisocyanate (IPDI) ;
   - Q⁴ représente CH2, CH2-CH2, CH2 monoalkoxylé, CH2-CH2 monoalkoxylé, CH2 polyalkoxylé ou CH2-CH2 polyalkoxylé.

Le monomère (f) peut également être choisi parmi les di(méth)acrylates comme le di(méth)acrylate de polyalkylène glycol, notamment le di(méth)acrylate de polypropylène glycol, le di(méth)acrylate d'éthylène glycol, le di(méth)acrylate de polyéthylène glycol, le di(méth)acrylate de triéthylène glycol, le di(méth)acrylate de 1,3-butylène glycol, le di(méth)acrylate de 1,6-butylène glycol, le di(méth)acrylate de 1,6-hexanediol, le di(méth)acrylate de néopentyl glycol, le di(méth)acrylate de 1,9-nonanediol, mais aussi le 2,2'-bis(4-(acryloxy-propyleoxyphényl))propane, le 2,2'-bis(4-(acryloxydiéthoxy-phényl))propane et l'acrylate de zinc; les composés tri(méth)acrylates tels que le tri(méth)acrylate de triméthylolpropane et le tri(méth)acrylate de triméthylolpropane éthoxylé, le tri(méth)acrylate triméthyloléthane, le tri(méth)acrylate pentaérythritol et le tri(méth)acrylate de tétraméthylolméthane ; les composés tétra(méth)acrylates tels que le tétra(méth)acrylate de ditriméthylolpropane, le tétra(méth)acrylate de tétraméthylolméthane et le tétra(méth)acrylate de pentaérythritol ; les composés hexa(méth)acrylates tels que l'hexa(méth)acrylate de dipentaérythritol ; les composés penta(méth)acrylates tels que le penta(méth)acrylate de dipentaérythritol ; les composés allyls tels que l'allyl (méth)acrylate, le diallylphthalate, l'itaconate de diallyl, le fumarate de diallyl, le maléate de diallyl ; les éthers polyallyls du sucrose ayant de 2 à 8 groupes par molécule, les éthers polyallyls du pentaérythritol tels que le pentaérythritol diallyl éther, le pentaérythritol triallyl éther et le pentaérythritol tetraallyl éther ; les éthers polyallyls du triméthylolpropane tels que l'éther diallyl triméthylolpropane et l'éther triallyl triméthylolpropane. D'autres composés polyinsaturés incluent le divinyl glycol, le divinyl benzène, le divinylcyclohexyl et le méthylènebisacrylamide.

Le monomère (f) peut également être préparé par une réaction d'estérification d'un polyol avec un anhydride insaturé tel que l'anhydride acrylique, l'anhydride méthacrylique, l'anhydride maléique ou l'anhydride itaconique. Pour obtenir le monomère (f), on peut également utiliser des composés choisis parmi les polyhaloalkanols tels que le 1,3-dichloroisopropanol et le 1,3-dibromoisopropanol ; les haloépoxyalkanes tels que l'épichlorohydrine, l'épibromohydrine, le 2-méthyle épichlorohydrine et l'épiiodohydrine ; polyglycidyls éthers tels que le 1,4-butanediol diglycidyl éther, glycérine-1,3-diglycidyl éther, éthylène glycol diglycidyl éther, propylène glycol diglycidyl éther, diéthylène glycol diglycidyl éther, néopentyl glycol diglycidyl éther, polypropylène glycol diglycidyl éther, bisphénol A-épichlorohydrine époxy résine et des mélanges.

Le monomère (f) peut également être choisi parmi les réticulants trifonctionnels. Il peut s'agir en particulier du tri(méth)acrylate de triméthylolpropane (TMPTA) ou du tri(méth)acrylate de triméthylolpropane éthoxylé (tel que par exemple le TMPTA 3OE).

Le monomère (f) peut également être choisi parmi le tri(méth)acrylate de triméthylolpropane, tri(méth)acrylate de triméthylolpropane éthoxylé, di(méth)acrylate d'éthylène glycol, méthylènebisacrylamide, diallylphtalate, diallylmaléate et leurs mélanges.

Le monomère (f) peut également être un mélange de deux monomères distincts, par exemple EGDCPEA (éthylène glycol dicyclopentényl éther acrylate) et TMPTA ou encore EGDCPEA et TMPTA 3OE.

Selon l'invention, le monomère (f) est, de préférence, choisi parmi un composé de formule (V), le triméthacrylate de triméthylolpropane, le triacrylate de triméthylolpropane, le triméthacrylate de triméthylolpropane éthoxylé, le triacrylate de triméthylolpropane éthoxylé, le diméthacrylate d'éthylène glycol, le diacrylate d'éthylène glycol, le méthylènebisacrylamide, le diallylphthalate, le diallylmaléate et leurs mélanges.

De manière également avantageuse, la réaction de polymérisation peut mettre en œuvre moins de 5 % molaire, de préférence de 0,01 à 4 % molaire, en particulier de 0,02 à 4 % molaire ou de 0,02 à 2 % molaire, notamment de 0,04 à 1 % molaire, de monomère (f) par rapport à la quantité molaire totale de monomères.

De manière également avantageuse, le copolymère (P1) peut être préparé par une réaction de polymérisation mettant également en œuvre au moins un monomère (g). Le monomère (g) est avantageusement un monomère réticulant, hydrophile, hydrophobe ou amphiphile et il est généralement un composé comprenant plusieurs insaturations éthyléniques. Il est distinct du monomère de formule (I) selon l'invention. Le monomère (g) peut être un composé de formule (VI) :
- un composé de formule de formule (VI) : 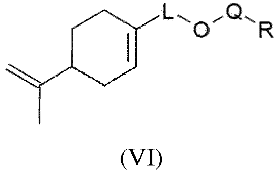 dans laquelle :
   - L représente CH₂, CH2 monoalkoxylé ou CH2 polyalkoxylé ;
   - Q représente une liaison directe ou C(O) ;
   - R représente -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ ou Q³OQ⁴OC(O)C(H)=CH₂ ;
   - Q³ représente un résidu divalent d'un composé diisocyanate dissymétrique, de préférence choisi parmi tolyl-1,3-diisocyanate (TDI) et isophorone-diisocyanate (IPDI) ;
   - Q⁴ représente CH2, CH2-CH2, CH2 monoalkoxylé, CH2-CH2 monoalkoxylé, CH2 polyalkoxylé ou CH2-CH2 polyalkoxylé ;
- un composé de formule (VI-A) : 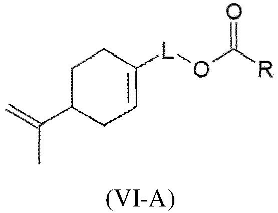 dans laquelle :
   - L représente CH2, CH2 monoalkoxylé ou CH2 polyalkoxylé ;
   - R représente -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ ou Q³OQ⁴OC(O)C(H)=CH₂ ;
   - Q³ représente un résidu divalent d'un composé diisocyanate dissymétrique, de préférence choisi parmi tolyl-1,3-diisocyanate (TDI) et isophorone-diisocyanate (IPDI) ;
   - Q⁴ représente CH2, CH2-CH2, CH2 monoalkoxylé, CH2-CH2 monoalkoxylé, CH2 polyalkoxylé ou CH2-CH2 polyalkoxylé ;
- un composé de formule (VI-B) : 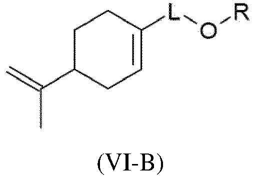 dans laquelle :
   - L représente CH2, CH2 monoalkoxylé ou CH2 polyalkoxylé ;
   - R représente -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ ou Q³OQ⁴OC(O)C(H)=CH₂ ;
   - Q³ représente un résidu divalent d'un composé diisocyanate dissymétrique, de préférence choisi parmi tolyl-1,3-diisocyanate (TDI) et isophorone-diisocyanate (IPDI) ;
   - Q⁴ représente CH2, CH2-CH2, CH2 monoalkoxylé, CH2-CH2 monoalkoxylé, CH2 polyalkoxylé ou CH2-CH2 polyalkoxylé.

Ces composés de formules (VI), (VI-A) et (VI-B) peuvent être préparés selon un procédé comprenant la réaction selon le schéma 1 au cours de laquelle la température est généralement comprise entre 50 et 250°C et qui peut mettre en œuvre un agent inhibiteur de radicaux.

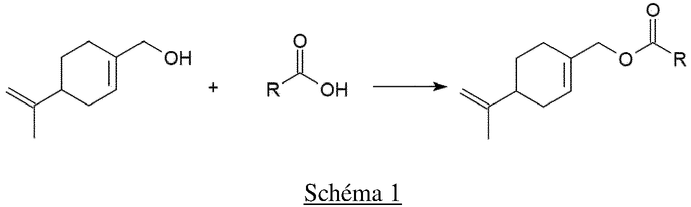

De manière également avantageuse, la réaction de polymérisation peut mettre en œuvre moins de 5 % molaire, de préférence de 0,01 à 4 % molaire, en particulier de 0,02 à 2 % molaire, notamment de de 0,04 à 1 % molaire, de monomère (g) par rapport à la quantité molaire totale de monomères.

Outre les différents monomères, la préparation du copolymère (P1) met également en œuvre au moins un agent de transfert de chaîne, de préférence choisi parmi les composés mercaptans, en particulier les composés mercaptans comprenant au moins quatre atomes de carbone tels que le butylmercaptan, le n-octylmercaptan, le n-dodécylmercaptan, le *tert*-dodécylmercaptan.

Le copolymère (P1) préparé selon l'invention est donc obtenu par une réaction de polymérisation. Cette réaction peut être une réaction de polymérisation radicalaire, par exemple une réaction de polymérisation en émulsion, en dispersion ou en solution. La polymérisation peut être conduite dans un solvant, en présence d'au moins un composé initiateur. Comme exemples de composés initiateurs, on connaît les sels de persulfate, notamment le persulfate d'ammonium.

De préférence, la réaction est une réaction de polymérisation radicalaire en émulsion. La polymérisation radicalaire en émulsion peut être réalisée en présence d'au moins un composé tensioactif et éventuellement d'au moins un agent de transfert de chaînes permettant généralement de réguler la masse moléculaire des chaînes produites lors de la polymérisation. Comme composés tensioactifs susceptibles d'être utilisés, on connaît :
- les tensioactifs anioniques, par exemple un sel d'acide gras, un sel alkylsulfate comme le laurylsulfate de sodium, un sel d'alkyléther sulfate comme le lauryl éther sulfate de sodium, un sel alkylbenzènesulfonate comme le dodécylbenzènesulfonate de sodium, un sel alkylphosphate ou un sel sulfosuccinate diester, un sel cocoamphoacétate comme le sodium cocoamphoacétate, un sel cocoamphodiacétate comme le sodium cocoamphodiacétate, un sel lauroyl glutamate comme le sodium lauroyl glutamate, un sel cocoyl isethionate comme le sodium cocoyl isethionate, un sel lauroyl méthyle isethionate comme le sodium lauroyl méthyle isethionate, un sel méthyle cocoyl taurate comme le sodium méthyle cocoyl taurate, un sel méthyle oleyl taurate comme le sodium méthyle oleyl taurate, un sel lauroyl sarcosinate comme le sodium lauroyl sarcosinate, un sel laureth 3 sulfosuccinate comme le sodium laureth 3 sulfosuccinate, un sel cocoyl apple amino acide comme le sodium cocoyl apple aminate, un sel cocoyl oat amino acide comme le sodium cocoyl oat aminate ;
- les tensioactifs non ioniques, par exemple un polyoxyéthylène alkyléther ou un ester d'acide gras polyoxyéthylène ;
- les tensioactifs cationiques, par exemple les halogénures d'alkyl-ammoniums quaternaires et les halogénures d'aryl-ammoniums quaternaires ;
- les tensioactifs zwitterioniques ou amphotères, par exemple les tensioactifs comprenant un groupe bétaïne ; et
- leurs mélanges.

L'invention concerne également l'utilisation, pour la préparation d'un copolymère (P1) défini selon l'invention, de moins de 5 % molaire par rapport à la quantité molaire totale de monomères mis en œuvre, d'au moins un monomère de formule (I) : 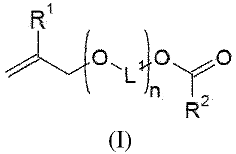 dans laquelle :
- R¹ représente indépendamment H ou CH3 ;
- R² représente indépendamment -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH;
- L¹ représente indépendamment un groupement éthylène, propylène ou butylène ;
- n représente indépendamment 0 ou un nombre entier ou décimal allant de 1 à 30.

Outre ce copolymère (P1) et son utilisation, l'invention concerne également un procédé de préparation de ce copolymère selon l'invention. Ainsi, l'invention fournit un procédé (1) de préparation d'un copolymère (P1) par réaction de polymérisation :
(a) d'au moins un monomère anionique comprenant au moins une insaturation éthylénique polymérisable, de préférence un monomère anionique comprenant une fonction vinylique polymérisable et au moins une fonction acide carboxylique ; et
(b) d'au moins un monomère non-ionique hydrophobe comprenant au moins une insaturation éthylénique polymérisable, de préférence un monomère non-ionique hydrophobe comprenant une fonction vinylique polymérisable et choisi parmi les acrylates de C₁-C₈-alkyle, les méthacrylates de C₁-C₈-alkyle, les maléates de C₁-C₈-alkyle, les itaconates de C₁-C₈-alkyle, les crotonates de C₁-C₈-alkyle, les cinnamates de C₁-C₈-alkyle et leurs mélanges ; et
(c) d'au moins un monomère de formule (I) : 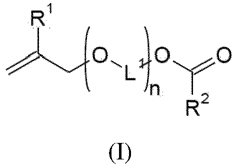 dans laquelle :
   - R¹ représente indépendamment H ou CH3 ;
   - R² représente indépendamment -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH;
   - L¹ représente indépendamment un groupement éthylène, propylène ou butylène ;
   - n représente indépendamment 0 ou un nombre entier ou décimal allant de 1 à 30.

Le procédé (1) selon l'invention est également défini par les monomères et par les conditions mis en œuvre pour la préparation du copolymère (P1) selon l'invention.

L'invention concerne également un procédé (2) de préparation d'un copolymère (P2) par réaction de polymérisation comprenant également la mise en œuvre lors de la réaction de polymérisation d'un copolymère (P1), obtenu préalablement lors de la réaction de polymérisation du procédé (1) selon l'invention.

Ainsi, le procédé (2) selon l'invention comprend la réaction de polymérisation d'un copolymère (P1) préparé préalablement selon l'invention avec :
(a) au moins un monomère anionique comprenant au moins une insaturation éthylénique polymérisable, de préférence un monomère anionique comprenant une fonction vinylique polymérisable et au moins une fonction acide carboxylique ; et
(b) au moins un monomère non-ionique hydrophobe comprenant au moins une insaturation éthylénique polymérisable, de préférence un monomère non-ionique hydrophobe comprenant une fonction vinylique polymérisable ; et
(c) au moins un monomère de formule (I) : 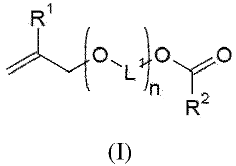 dans laquelle :
   - R¹ représente indépendamment H ou CH3 ;
   - R² représente indépendamment -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH ;
   - L¹ représente indépendamment un groupement éthylène, propylène ou butylène ;
   - n représente indépendamment 0 ou un nombre entier ou décimal allant de 1 à 30.

Le procédé (2) selon l'invention est également défini par les monomères et par les conditions mis en œuvre pour la préparation du copolymère (P1) selon l'invention.

De manière avantageuse, le copolymère (P2) multiphasique selon l'invention peut être préparé de façon séquentielle, par polymérisation radicalaire en émulsion, en dispersion ou en solution. De préférence, au moins deux étapes consécutives sont mises en œuvre, une première étape permettant l'obtention d'un premier copolymère (P1), tandis qu'une deuxième étape de polymérisation met en œuvre un copolymère (P1).

Pratiquement, la première étape consiste à mettre en contact les monomères de préparation du copolymère (P1) avec un composé initiateur de polymérisation. Cette mise en contact peut être réalisée en mode discontinu, en mode batch, encore en mode semi-batch ou en mode semi-continu. Cette mise en contact peut être réalisée en une durée pouvant aller de plusieurs minutes à plusieurs heures.

La deuxième étape de préparation du copolymère (P2) peut comprendre :
- l'ajout des monomères de préparation du copolymère (P2) à un milieu de dispersion comprenant le copolymère (P1) déjà formé, par exemple selon un mode discontinu, un mode batch, un mode semi-batch ou un mode semi-continu, et selon une durée pouvant aller de plusieurs minutes à plusieurs heures, et
- simultanément pour le mode semi-continu ou postérieurement pour le mode discontinu, l'introduction d'un composé initiateur de polymérisation.

Cette étape permet alors la formation d'un copolymère (P2) selon l'invention.

L'invention concerne donc également le copolymère (P2) pouvant être obtenu selon le procédé (2) selon l'invention.

De manière avantageuse, le copolymère (P2) selon l'invention est multiphasique. De manière également avantageuse, le copolymère (P2) selon l'invention comprend un cœur comprenant un premier copolymère (P1), totalement ou partiellement recouvert par un deuxième copolymère (P1), identique ou différent du premier copolymère (P1).

De manière préférée, le copolymère (P2) selon l'invention comprend un cœur comprenant un premier copolymère (P1), totalement ou partiellement recouvert par un deuxième copolymère (P1) pour lequel le rapport pondéral 1° copolymère (P1)/2° copolymère (P1) est compris entre 45/55 et 95/5, en particulier entre 60/40 et 90/10.

Les copolymères selon l'invention se révèlent particulièrement efficaces à titre d'agents modificateurs de rhéologie dans une large gamme de compositions aqueuses ou encore en tant qu'agent épaississant et suspensif. On peut citer les compositions aqueuses dans de nombreux domaines industriels et notamment les fluides de fracking en forage, les formulations pour la céramique, les sauces de couchage papetière. On cite en particulier des compositions lavantes renfermant des tensioactifs, telles que des compositions de soin ou des compositions d'entretien («personal care» et «home care» en anglais) comprenant par exemple des compositions cosmétiques, d'hygiène de la personne, des produits de toilette et compositions nettoyantes pour une application sur le corps (incluant la peau, les cheveux, les ongles) des humains ou des animaux, par exemple des compositions de shampoing, ou encore des compositions utilisées pour le nettoyage ou le maintien des conditions sanitaires, par exemple dans la cuisine, la salle de bain, les produits détergents, les produits de lessive, etc.

Les copolymères (P1) et (P2) selon l'invention possèdent des propriétés avantageuses. Ils peuvent donc être intégrés à des compositions aqueuses. Ainsi, l'invention fournit également une composition aqueuse comprenant au moins un copolymère (P1) selon l'invention. L'invention fournit également une composition aqueuse comprenant au moins un copolymère (P2) selon l'invention. L'invention fournit également une composition aqueuse comprenant au moins un copolymère (P1) selon l'invention et au moins un copolymère (P2) selon l'invention.

De manière préférée, la composition aqueuse selon l'invention est une composition cosmétique et peut comprendre :
- au moins un copolymère (P1) selon l'invention ; ou
- au moins un copolymère (P2) selon l'invention ; ou
- au moins un copolymère (P1) selon l'invention et au moins un copolymère (P2) selon l'invention.

Au sein de la composition selon l'invention, les copolymères (P1) et (P2) selon l'invention peuvent être présents en des quantités allant de 0,1 % à 20 % en poids, en particulier de 0,5 % à 12 % en poids, par rapport au poids total de la composition.

Outre un copolymère selon l'invention, la composition selon l'invention peut comprendre une phase continue limpide et des particules en suspension réparties dans la phase continue. Le copolymère selon l'invention peut alors conférer à la composition, clarté et maintien en suspension des particules présentes.

Au moment de son utilisation, une telle composition selon l'invention ne nécessite généralement aucune étape de mélange, même si la composition a été stockée plusieurs semaines, voire plusieurs mois.

La composition selon l'invention peut également comprendre un ou plusieurs composés tensioactifs, en particulier choisis parmi les tensioactifs anioniques, zwitterioniques ou amphotères, cationiques ou non-ioniques, et leurs mélanges. Elle peut également comprendre un ou plusieurs ingrédients actifs.

De manière plus préférée, la composition cosmétique selon l'invention a un pH allant de 3 à 9. De manière encore plus préférée, son pH va de 3 à 7. Encore plus préférentiellement, son pH va de 4 à 7.

L'invention concerne donc l'utilisation pour la préparation d'une composition aqueuse selon l'invention ou pour la préparation d'une composition cosmétique selon l'invention :
- d'au moins un copolymère (P1) selon l'invention ; ou
- d'au moins un copolymère (P2) selon l'invention ; ou
- d'au moins un copolymère (P1) selon l'invention et d'au moins un copolymère (P2) selon l'invention.

### Exemples :

Les exemples qui suivent permettent d'illustrer les différents aspects de l'invention. Les abréviations suivantes sont utilisées :
- AMA : acide méthacrylique,
- AE : acrylate d'éthyle,
- SR 351 de Sartomer : triacrylate de triméthylolpropane (TMPTA),
- SIPOMER HPM100 de Solvay-Rhodia : méthacrylate de nopol 10 OE,
- Polyglykol B11/50 de Clariant : éthylène oxide-propylène oxide-monobutyl éther,
- Empicol LXVN de Huntsmann : laurylsulfate de sodium (SDS),
- Texapon NS0 de BASF : laureth sulfate d'ammonium en solution à 28 % ou lauryl éther sulfate d'ammonium en solution à 28 % (SLES),
- persulfate de sodium (NH₄)₂S₂O₈,
- composé (d) : solution comprenant 45 % en poids d'acide méthacrylique, 5 % en poids d'eau et 50 % en poids de composé (d2-1) de formule (II) dans laquelle T¹ représente un groupement -OC(O)C(CH₃)=CH₂, T² représente une chaîne hydrocarbonée ramifiée comportant 16 atomes de carbone (2-hexyldécanyl), m représente 25 et p et q représentent 0.

### Préparation des polymères selon un procédé semi-batch :

Les réactifs et quantités mis en œuvre sont présentés dans le tableau 1.

Dans un réacteur de 1 L agité et chauffé à l'aide d'un bain d'huile, on prépare le mélange 1 en introduisant de l'eau désionisée et une solution contenant 28 % massique de lauryl éther sulfate de sodium (SLES) ou du laurylsulfate de sodium (SDS), et éventuellement de l'éthylène oxide-propylène oxide-monobutyl éther.

On prépare dans un bécher un mélange 2 dit pré-mélange comprenant :
- acide méthacrylique,
- acrylate d'éthyle,
- composé (c), TMPTA ou Sipomer HPM100,
- éventuellement composé (d),
- éventuellement eau désionisée,
- éventuellement solution à 28 % de lauryl éther sulfate de sodium (SLES) ou du laurylsulfate de sodium (SDS),
- éventuellement éthylène oxide-propylène oxide-monobutyl éther.

### Ce pré-mélange est agité afin de former un mélange monomériques.

De manière analogue, on prépare un pré-mélange comparatif ne comprenant pas de composé (c).

On prépare une solution d'initiateur 1 comprenant du persulfate d'ammonium et de l'eau désionisée. On prépare une solution d'initiateur 2 comprenant également du persulfate d'ammonium et de l'eau désionisée.

Dans le réacteur chauffé à la température de 85°C ± 1°C, on injecte en parallèle durant 2 heures, la solution d'initiateur 1 ainsi que le pré-mélange de monomères. Puis durant 1 heure, on injecte la solution d'initiateur 2 dans le réacteur chauffé à 85°C ± 1°C.

On ajoute éventuellement de l'eau et on cuit 30 min à la température de 85°C +/- 1°C. L'ensemble est ensuite refroidi à température ambiante.

Les polymères selon l'invention et les polymères comparatifs ont été préparés dans ces conditions en faisant varier les compositions de monomères des pré-mélanges de monomères.

Les compositions des copolymères obtenus sont présentées dans le tableau 1.

**Tableau 1**

| | | 1 | 2 | 3 | 4 | 5 | 6 | 7 | 8 | 9 | 10 | 11 | 12 |
|---|---|---|---|---|---|---|---|---|---|---|---|---|---|
| | | exemple | | | | | | | | | | | |
| | | selon l'invention | | | | | | | | | comparatif | | |
| composé (c) | | (c1) | (c1) | (c1) | (c1) | (c2) | (c2) | (c1) | (c1) | (c1) | 0 | TMPTA | Sipomer HPM100 |
| mélange 1 | eau | 400 | 400 | 400 | 400 | 400 | 400 | 400 | 400 | 432 | 432 | 432 | 400 |
| | SDS | 2,6 | 2,6 | 2,6 | 2,6 | 2,6 | 2,6 | 2,6 | 2,6 | 0 | 0 | 0 | 2,6 |
| | SLES | 0 | 0 | 0 | 0 | 0 | 0 | 0 | 0 | 9,29 | 9,29 | 9,29 | 0 |
| | B11/50 | 1,1 | 1,1 | 1,1 | 1,1 | 1,1 | 1,1 | 1,1 | 1,1 | 0 | 0 | 0 | 1,1 |
| mélange 2 | eau | 173,7 | 173,7 | 173,7 | 173,7 | 173,7 | 173,7 | 173,7 | 173,7 | 26,00 | 26,06 | 172,50 | 173,7 |
| | SDS | 1,81 | 1,81 | 1,81 | 1,81 | 1,81 | 1,81 | 1,81 | 1,81 | 0 | 0 | 0 | 1,81 |
| | SLES | 0 | 0 | 0 | 0 | 0 | 0 | 0 | 0 | 0 | 0 | 6,47 | 0 |
| | B11/50 | 1,04 | 1,04 | 1,04 | 1,04 | 1,04 | 1,04 | 1,04 | 1,04 | 0 | 0 | 0 | 1,04 |
| | composé (a) AMA | 105,69 | 105,69 | 105,69 | 105,69 | 105,69 | 105,69 | 105,69 | 99,74 | 76,31 | 76,31 | 76,66 | 99,74 |
| | composé (b) AE | 191,28 | 191,28 | 191,28 | 191,28 | 191,28 | 191,28 | 191,28 | 191,28 | 196,10 | 196,10 | 196,82 | 191,28 |
| | composé (c) | 2,75 | 0,83 | 1,925 | 2,33 | 1,925 | 1,1 | 3,44 | 2,75 | 1,10 | 0,00 | 1,00 | 2,75 |
| | composé (d2-1) | 0 | 0 | 0 | 0 | 0 | 0 | 0 | 12,8 | 51,91 | 51,91 | 51,91 | 12,8 |
| initiateur 1 | (NH₄)₂S₂O₈ | 0,587 | 0,587 | 0,587 | 0,587 | 0,587 | 0,587 | 0,587 | 0,587 | 0,467 | 0,467 | 0,467 | 0,587 |
| | eau | 58 | 59 | 60 | 61 | 65 | 67 | 62 | 57 | 55 | 55 | 55 | 55 |
| initiateur 2 | (NH₄)₂S₂O₈ | 0,123 | 0,123 | 0,123 | ^{∗}0,123 | 0,123 | 0,123 | 0,123 | 0,123 | 0,123 | 0,123 | 0,123 | 0,123 |
| | eau | 40 | 40 | 40 | 40 | 40 | 40 | 40 | 40 | 20 | 20 | 20 | 40 |
| rinçage ou ajustement | eau | 20 | 20 | 20 | 20 | 20 | 20 | 20 | 20 | 11,25 | 15 | 15 | 20 |
| Composition massique du polymère (%) | résidu composé (a) AMA | 35,26 | 35,49 | 35,36 | 35,31 | 35,36 | 35,46 | 35,18 | 34,55 | 31,11 | 31,21 | 31,12 | 34,55 |
| | résidu composé (b) AE | 63,82 | 64,23 | 64,00 | 63,91 | 64,00 | 64,17 | 63,67 | 62,52 | 60,73 | 60,94 | 60,77 | 62,52 |
| | résidu composé (c) | 0,92 | 0,28 | 0,64 | 0,78 | 0,64 | 0,37 | 1,15 | 0,90 | 0,34 | 0,00 | 0,31 | 0,90 |
| | résidu composé (d2-1) | 0,00 | 0,00 | 0,00 | 0,00 | 0,00 | 0,00 | 0,00 | 2,04 | 7,82 | 7,85 | 7,80 | 2,04 |
| Composition molaire du polymère (%) | résidu composé (a) AMA | 39,05 | 39,10 | 39,07 | 39,06 | 39,03 | 39,07 | 39,04 | 39,00 | 37,09 | 37,12 | 37,07 | 39,01 |
| | résidu composé (b) AE | 60,79 | 60,85 | 60,82 | 60,80 | 60,75 | 60,81 | 60,76 | 60,70 | 62,27 | 62,31 | 62,25 | 60,72 |
| | résidu composé (c) | 0,16 | 0,05 | 0,11 | 0,13 | 0,22 | 0,12 | 0,20 | 0,16 | 0,06 | 0,00 | 0,11 | 0,13 |
| | résidu composé (d2-1) | 0,00 | 0,00 | 0,00 | 0,00 | 0,00 | 0,00 | 0,00 | 0,14 | 0,57 | 0,57 | 0,57 | 0,14 |

### Evaluation des propriétés des polymères au sein d'une formulation aqueuse :

La formulation aqueuse mise en œuvre comprend 2,4 % ou 3 % en poids de polymère (voir tableau 1), 9 % en poids d'un premier composé tensio-actif (SLES ou lauryl éther sulfate de sodium), 3 % en poids d'un deuxième composé tensio-actif (CAPB ou cocamidopropyle bétaïne) et de l'eau (qsp 100 % en poids). Le pH de la formulation est ajusté à une valeur de 5, 6 ou 7 par ajout d'acide lactique ou d'hydroxyde de sodium.

Les formulations sont évaluées pour leurs propriétés de viscosité, de clarté et de performances suspensivantes.

### Viscosité :

On mesure la viscosité des formulations à l'aide d'un viscosimètre Brookfield, modèle LVT. Avant la mesure de la viscosité, on laisse chacune des formulations au repos durant 24 heures à 25°C. Le mobile du viscosimètre doit être centré par rapport à l'ouverture du flacon de formulation. On mesure la viscosité à 6 tours par minute à l'aide du module approprié. On laisse tourner le viscosimètre jusqu'à ce que la valeur de viscosité soit stabilisée.

Le copolymère agent modificateur de rhéologie doit apporter une viscosité suffisante à la formulation dans laquelle il est mis en œuvre. D'une manière générale, la viscosité souhaitée pour les formulations épaissies devrait être supérieure à 4 000 mPa.s, en particulier supérieure à 6 000 mPa.s et plus particulièrement supérieure à 8 000 mPa.s.

### Clarté :

La clarté de chaque formulation est évaluée par mesure de la transmittance au moyen d'un spectromètre UV Genesys 10 UV (Cole Parmer), équipé de cuves Rotilabo-Einmal Kuvetten PS, 4,5 mL. On préchauffe l'appareil durant 10 minutes avant utilisation puis on réalise une première mesure au moyen d'une cuve remplie de 3,8 mL d'eau bipermutée. On réalise ensuite une mesure avec une cuve remplie de 3,8 mL de formulation cosmétique à tester. La transmittance est mesurée à la longueur d'onde de 500 nm. Plus la valeur de transmittance, exprimée en pourcentage, est élevée, plus la composition cosmétique est limpide. Pour une valeur de transmittance à 500 nm d'au moins 60 % la formulation est limpide.

### Performances suspensivantes :

Des mesures de viscoélasticité sont réalisées sur les formulations à l'aide d'un Rhéomètre de type Haake - Mars III. Les variations de Tan(δ) et de G' en fonction de la contrainte τ (balayage de 0 à 1 000 dyn/cm²) sont mesurées à 25°C à l'aide d'une géométrie cône / plan 1°. Les valeurs de Tan(δ), de G' à 10 dyn/cm² et la valeur de résistance élastique sont déduites de cette mesure.

En règle générale, les formulations ont de bonnes propriétés suspensivantes pour des valeurs combinées de G' > 50 Pa, de Tan(δ) < 0,55 et de résistance élastique > 70 dyn/cm².

Les résultats obtenus sont présentés dans le tableau 2.

On constate que le copolymère selon l'invention permet de combiner avantageusement des performances en termes d'effet épaississant, de clarté et de propriétés suspensivantes. Autrement dit, il permet l'obtention d'une formulation aqueuse présentant la viscosité souhaitée et comprenant une phase continue limpide et des particules en suspension réparties de manière homogène dans la phase continue.

**Tableau 2**

| | | 1 | 2 | 3 | 4 | 5 | 6 | 7 | 8 | 9 | 10 | 11 | 12 |
|---|---|---|---|---|---|---|---|---|---|---|---|---|---|
| | | exemple | | | | | | | | | | | |
| | | selon l'invention | | | | | | comparatif | | | | | |
| Résultats applicatifs à 3 % (ou ^{∗} à 2,4 % et à pH = 5) | viscosité Brookfield (mPa.s) pH = 5 | 13 900 | 13 340 | 12 800 | 10 060 | 8 200 | 13560 | 11 280 | 19 080 | 17 160^{∗} | 5 920^{∗} | 8 000^{∗} | 2 970 |
| | viscosité Brookfield (mPa.s) pH = 6 | 26 300 | 19 600 | 21 800 | 25 690 | 19 400 | 21 700 | 24 190 | 29800 | NA | NA | NA | 1 530 |
| | viscosité Brookfield (mPa.s) pH = 7 | 7 000 | 9 380 | 12 420 | 10 180 | 11 000 | 9 880 | 6 600 | 13 840 | NA | NA | NA | 20 100 |
| | Tan (delta) pH = 7 (^{∗} à pH = 5) | 0,33 | 0,37 | 0,33 | 0,32 | 0,24 | 0,35 | 0,33 | 0,39 | 0,36^{∗} | 1,33^{∗} | 0,67^{∗} | 1,89 |
| | T à 500 nm (%) pH = 7 (^{∗} à pH = 5) | 97 | 96 | 98 | 97 | 92 | 97 | 94 | 98 | 94^{∗} | 98^{∗} | 94^{∗} | 99 |
| | G' (Pa) pH = 7 (^{∗} à pH = 5) | 52 | 79 | 95 | 84 | 107 | 86 | 64 | 102 | 71^{∗} | 5^{∗} | 30^{∗} | 31 |
| | résistance élastique (dyn/cm²) pH = 7 (^{∗} à pH=5) | | 96 | 95 | 95 | 96 | 96 | NA | 119 | 93^{∗} | 0^{∗} | 229^{∗} | 0 |

## Revendications

1. Copolymère (P1) obtenu par réaction de polymérisation :
(a) d'au moins un monomère anionique comprenant au moins une insaturation éthylénique polymérisable ; et
(b) d'au moins un monomère non-ionique hydrophobe comprenant au moins une insaturation éthylénique polymérisable et choisi parmi les acrylates de C₁-C₈-alkyle, les méthacrylates de C₁-C₈-alkyle, les maléates de C₁-C₈-alkyle, les itaconates de Ci-Cs-alkyle, les crotonates de C₁-C₈-alkyle, les cinnamates de C₁-C₈-alkyle et leurs mélanges ; et
(c) d'au moins un monomère de formule (I) : 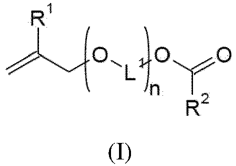 dans laquelle :
• R¹ représente indépendamment H ou CH3 ;
• R² représente indépendamment -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH ;
• L¹ représente indépendamment un groupement éthylène, propylène ou butylène ;
• n représente indépendamment 0 ou un nombre entier ou décimal allant de 1 à 30.

2. Copolymère selon la revendication 1
• pour lequel le monomère anionique (a) est un monomère anionique comprenant une fonction vinylique polymérisable et au moins une fonction acide carboxylique, de préférence un monomère choisi parmi l'acide acrylique, l'acide méthacrylique, l'acide maléique, l'acide itaconique, l'acide crotonique, un sel d'acide acrylique, un sel d'acide méthacrylique, un sel d'acide maléique, un sel d'acide itaconique, un sel d'acide crotonique, un sel d'acide cinnamique, et leurs mélanges ; ou
• obtenu par réaction de polymérisation mettant en œuvre au moins 20 % molaire, de préférence de 25 à 60 % molaire, en particulier de 30 à 55 % molaire, de monomère anionique (a) par rapport à la quantité molaire totale de monomères.

3. Copolymère selon l'une des revendications 1 et 2
• pour lequel le monomère non-ionique hydrophobe (b) est un monomère non-ionique hydrophobe comprenant une fonction vinylique polymérisable choisi parmi l'acrylate de méthyle, l'acrylate d'éthyle, l'acrylate de butyle, l'acrylate de 2-éthyle-hexyle, le méthacrylate de méthyle, le méthacrylate d'éthyle, le méthacrylate de butyle et leurs mélanges ; ou
• obtenu par réaction de polymérisation mettant en œuvre de 30 à 80 % molaire, de préférence de 35 à 75 % molaire plus préférentiellement de 45 à 70 % molaire, de monomère non-ionique hydrophobe (b) par rapport à la quantité molaire totale de monomères.

4. Copolymère selon l'une des revendications 1 à 3
• pour lequel le monomère (c) est choisi parmi un composé (c1) de formule (I) dans laquelle R¹ représente H ; R² représente -C(H)=CH₂ ; L¹ représente CH2-CH2 et n représente 10 ; un composé (c2) de formule (I) dans laquelle R¹ représente H ; R² représente -C(CH₃)=CH_{2;} L¹ représente CH2-CH2 et n représente 3,5 ; un composé (c3) de formule (I) dans laquelle R¹ représente H ; L¹ représente CH₂-CH_{2;} R² représente -C(CH₃)=CH₂ et n représente 10 ; ou
• obtenu par réaction de polymérisation mettant en œuvre de moins de 5 %, de préférence de 0,01 à 5 % molaire et plus préférentiellement de 0,02 à 4 % ou de 0,02 à 2 % molaire ou de 0,04 à 0,5 % molaire de 0,04 à 0,25 % molaire, de monomère (c) par rapport à la quantité molaire totale de monomères.

5. Copolymère selon l'une des revendications 1 à 4 obtenu par réaction de polymérisation mettant également en œuvre au moins un monomère non-ionique (d), différent du monomère (b), comprenant une fonction vinylique polymérisable et une chaîne hydrocarbonée comprenant au moins 10 atomes de carbone, de préférence choisi parmi :
• un monomère (d1) comprenant une fonction vinylique polymérisable et une chaîne hydrocarbonée en C₁₂-C₃₆ ;
• un monomère (d2) comprenant une fonction vinylique polymérisable, une chaîne hydrocarbonée en C₁₂-C₃₆ et de 1 à 150, de préférence de 15 à 50 et plus préférentiellement de 20 à 30, groupements alkylène-oxy, de préférence un monomère (d2) de formule (II) :
T¹-(CH₂CH₂O)ₘ-(CH₂CH(CH₃)O)ₚ-(CH₂CH(CH₂CH₃)O)_{q}-T² (II)
dans laquelle :
o T¹ représente indépendamment une fonction vinylique polymérisable ;
o T² représente indépendamment :
▪ une chaîne hydrocarbonée comprenant au moins 10 atomes de carbone, de préférence une chaîne hydrocarbonée en C₁₂-C₃₆ ; ou
▪ une chaîne hydrocarbonée comprenant au moins 10 atomes de carbone, de préférence une chaîne hydrocarbonée en C₁₂-C₃₆, et au moins un hétéroatome choisi parmi O, S, N et P ; ou
o m, p et q, identiques ou différents, représentent indépendamment un nombre entier ou décimal allant de 0 à 150, la somme de m, p et q étant non-nulle ;
de préférence obtenu par réaction de polymérisation mettant en œuvre de 0,01 à 10 % molaire ou de 0,02 à 5 % molaire ou de 0,02 à 2 % molaire, de monomère (d) par rapport à la quantité molaire totale de monomères.

6. Copolymère selon l'une des revendications 1 à 5 obtenu par réaction de polymérisation mettant également en œuvre au moins un monomère ionique ou non-ionique (e), différent du monomère (b), choisi parmi :
- l'acide 2-acrylamido-2-méthylpropane sulfonique ou un de ses sels ;
- les télomères, de préférence les dimères, les trimères ou les tétramères, insaturés de l'acide acrylique ;
- les monomères de formule (III) : 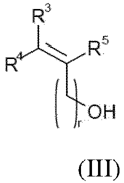 dans laquelle :
▪ R³, R⁴ et R⁵, identiques ou différents, représentent indépendamment H ou CH3 ;
▪ r représente indépendamment 1, 2 ou 3 ;
- les monomères de formule (IV) , notamment HEMA, HPMA et HPA : 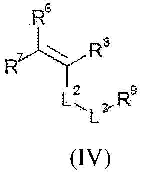 dans laquelle :
▪ R⁶, R⁷ et R⁸, identiques ou différents, représentent indépendamment H ou CH3 ;
▪ R⁹ représente H ou CH3 ;
▪ L² représente indépendamment une liaison directe ou un groupement choisi parmi O, C(O)O, CH2CH2 et CH2 ;
▪ L³ représente indépendamment une liaison directe ou de 1 à 150, de préférence de 15 à 50 et plus préférentiellement de 25 à 30, groupements alkylène-oxy ;
de préférence obtenu par réaction de polymérisation mettant en œuvre de 0,01 à 25 % molaire ou de 0,02 à 15 % molaire ou de 0,02 à 10 % molaire, de monomère (e) par rapport à la quantité molaire totale de monomères.

7. Copolymère selon l'une des revendications 1 à 6 obtenu par réaction de polymérisation mettant également en œuvre au moins un autre monomère (f), de préférence choisi parmi un composé de formule (V), le triméthacrylate de triméthylolpropane, le triacrylate de triméthylolpropane, le triméthacrylate de triméthylolpropane éthoxylé, le triacrylate de triméthylolpropane éthoxylé, le diméthacrylate d'éthylène glycol, le diacrylate d'éthylène glycol, le méthylènebisacrylamide, le diallylphthalate, le diallylmaléate et leurs mélanges : 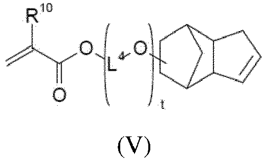 dans laquelle :
• R¹⁰ représente indépendamment H ou CH3 ;
• L⁴ représente indépendamment un groupement C₁-C₂₀-alkylène linéaire ou ramifié ;
• t représente indépendamment 0 ou un nombre entier allant de 1 à 30, par exemple de 1 à 20, en particulier de 1 à 15, notamment de 1 à 10 ;
de préférence obtenu par réaction de polymérisation mettant en œuvre moins de 5 % molaire, de préférence de 0,01 à 4 % molaire, en particulier de 0,02 à 2 % molaire, notamment de de 0,04 à 1 % molaire, de monomère (f) par rapport à la quantité molaire totale de monomères.

8. Copolymère selon l'une des revendications 1 à 7 obtenu par réaction de polymérisation mettant également en œuvre au moins un autre monomère (g) de formule
• un composé de formule de formule (VI) : 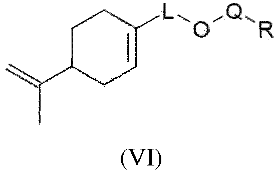 dans laquelle :
- L représente CH2, CH2 monoalkoxylé ou CH2 polyalkoxylé ;
- Q représente une liaison directe ou C(O) ;
- R représente -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ ou Q³OQ⁴OC(O)C(H)=CH₂ ;
- Q³ représente un résidu divalent d'un composé diisocyanate dissymétrique, de préférence choisi parmi tolyl-1,3-diisocyanate (TDI) et isophorone-diisocyanate (IPDI) ;
- Q⁴ représente CH2, CH2-CH2, CH2 monoalkoxylé, CH2-CH2 monoalkoxylé, CH2 polyalkoxylé ou CH2-CH2 polyalkoxylé ;
• un composé de formule (VI-A) : 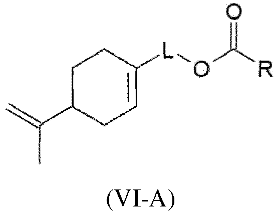 dans laquelle :
- L représente CH₂, CH₂ monoalkoxylé ou CH₂ polyalkoxylé ;
- R représente -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ ou Q³OQ⁴OC(O)C(H)=CH₂ ;
- Q³ représente un résidu divalent d'un composé diisocyanate dissymétrique, de préférence choisi parmi tolyl-1,3-diisocyanate (TDI) et isophorone-diisocyanate (IPDI) ;
- Q⁴ représente CH2, CH2-CH2, CH2 monoalkoxylé, CH2-CH2 monoalkoxylé, CH2 polyalkoxylé ou CH2-CH2 polyalkoxylé ;
• un composé de formule (VI-B) : 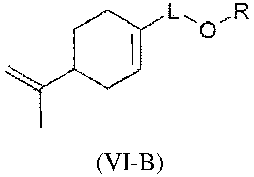 dans laquelle :
- L représente CH2, CH2 monoalkoxylé ou CH2 polyalkoxylé ;
- R représente -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ ou Q³OQ⁴OC(O)C(H)=CH₂;
- Q³ représente un résidu divalent d'un composé diisocyanate dissymétrique, de préférence choisi parmi tolyl-1,3-diisocyanate (TDI) et isophorone-diisocyanate (IPDI) ;
- Q⁴ représente CH2, CH2-CH2, CH2 monoalkoxylé, CH2-CH2 monoalkoxylé, CH2 polyalkoxylé ou CH2-CH2 polyalkoxylé ;
de préférence obtenu par réaction de polymérisation mettant en œuvre moins de 5 % molaire, de préférence de 0,01 à 4 % molaire, en particulier de 0,02 à 4 % molaire ou de 0,02 à 2 % molaire, notamment de de 0,04 à 1 % molaire, de monomère (g) par rapport à la quantité molaire totale de monomères.

9. Utilisation, pour la préparation d'un copolymère (P1) défini selon l'une des revendications précédentes, de moins de 5 % molaire par rapport à la quantité molaire totale de monomères, d'au moins un monomère de formule (I) : 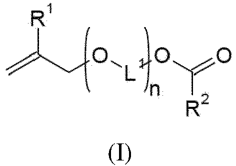 dans laquelle :
• R¹ représente indépendamment H ou CH3 ;
• R² représente indépendamment -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH ;
• L¹ représente indépendamment un groupement éthylène, propylène ou butylène ;
• n représente indépendamment 0 ou un nombre entier ou décimal allant de 1 à 30.

10. Procédé (1) de préparation d'un copolymère (P1) par réaction de polymérisation :
(a) d'au moins un monomère anionique comprenant au moins une insaturation éthylénique polymérisable, de préférence un monomère anionique comprenant une fonction vinylique polymérisable et au moins une fonction acide carboxylique ; et
(b) d'au moins un monomère non-ionique hydrophobe comprenant au moins une insaturation éthylénique polymérisable, choisi parmi les acrylates de C₁-C₈-alkyle, les méthacrylates de C₁-C₈-alkyle, les maléates de C₁-C₈-alkyle, les itaconates de Ci-Cs-alkyle, les crotonates de C₁-C₈-alkyle, les cinnamates de C₁-C₈-alkyle et leurs mélanges ; et
(c) d'au moins un monomère de formule (I): 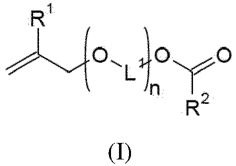 dans laquelle :
• R¹ représente indépendamment H ou CH3 ;
• R² représente indépendamment -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH;
• L¹ représente indépendamment un groupement éthylène, propylène ou butylène ;
• n représente indépendamment 0 ou un nombre entier ou décimal allant de 1 à 30.

11. Procédé (2) de préparation d'un copolymère (P2) par réaction de polymérisation définie selon la revendication 10 et comprenant également la mise en œuvre, lors de la réaction de polymérisation, d'un copolymère (P1), obtenu préalablement par réaction de polymérisation selon les revendications 1 à 8.

12. Copolymère (P2) pouvant être obtenu selon le procédé (2) de la revendication 11, de préférence un copolymère (P2) multiphasique ou bien qui comprend un cœur comprenant un premier copolymère (P1), totalement ou partiellement recouvert par un deuxième copolymère (P1), identique ou différent du premier copolymère (P1) ; en particulier pour lequel le rapport pondéral 1°copolymère (P1)/2°copolymère (P1) est compris entre 45/55 et 95/5, en particulier entre 60/40 et 90/10.

13. Composition aqueuse comprenant au moins un copolymère (P1) selon l'une des revendications 1 à 8 ou au moins un copolymère (P2) selon la revendication 12.

14. Composition cosmétique, en particulier composition cosmétique dont le pH va de 3 à 9, de préférence de 3 à 7, plus préférentiellement de 4 à 7, comprenant :
• au moins un copolymère (P1) selon l'une des revendications 1 à 8 ; ou
• au moins un copolymère (P2) selon la revendication 12 ; ou
• au moins un copolymère (P1) selon l'une des revendications 1 à 8 et au moins un copolymère (P2) selon la revendication 12 ; ou
• au moins une composition aqueuse définie selon la revendication 13.

15. Utilisation pour la préparation d'une composition aqueuse selon la revendication 13 ou pour la préparation d'une composition cosmétique selon la revendication 14,
• d'au moins un copolymère (P1) selon l'une des revendications 1 à 8 ; ou
• d'au moins un copolymère (P2) selon la revendication 12 ; ou
• d'au moins un copolymère (P1) selon l'une des revendications 1 à 8 et d'au moins un copolymère (P2) selon la revendication 12.

## Patentansprüche

1. Copolymer (P1), hergestellt durch Polymerisationsreaktion:
(a) mindestens eines anionischen Monomers mit mindestens einer polymerisierbaren ethylenischen Ungesättigtheit; und
(b) mindestens eines nicht-ionischen hydrophoben Monomers mit mindestens einer polymerisierbaren ethylenischen Ungesättigtheit und ausgewählt aus C₁-C₈-Alkylacrylaten, C₁-C₈-Alkylmethacrylaten, C₁-C₈-Alkylmaleaten, C₁-C₈-Alkylitaconaten, de C₁-C₈-Alkylcrotonaten, C₁-C₈-Alkylcinnamaten und Mischungen davon; und
(c) mindestens eines Monomers mit der Formel (I): 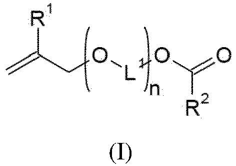 wobei:
• R¹ unabhängig H oder CH3 darstellt;
• R² unabhängig -C(H)=CH2, -C(CH₃)=CH₂, -C(H)=C(H)C(O)OH, -C(H)=C(H)CH₃, -C(=CH₂)CH₂C(O)OH, -CH₂C(=CH₂)C(O)OH darstellt;
• L¹ unabhängig eine Ethylen-, Propylen- oder Butylen-Gruppe darstellt;
• n unabhängig 0 oder eine Ganz- bzw. Dezimalzahl von 1 bis 30 darstellt.

2. Copolymer nach Anspruch 1,
• wobei das anionische Monomer (a) einem anionischen Monomer mit einer polymerisierbaren funktionellen Vinylgruppe und mindestens einer Carbonsäure-Funktion entspricht, vorzugsweise einem Monomer ausgewählt aus Acrylsäure, Methacrylsäure, Maleinsäure, Itaconsäure, Crotonsäure, einem Salz der Acrylsäure, einem Salz der Methacrylsäure, einem Salz der Maleinsäure, einem Salz der Itaconsäure, einem Salz der Crotonsäure, einem Salz der Zimtsäure und Mischungen davon; oder
• das durch Polymerisationsreaktion mit Umsetzung von mindestens 20 Mol-%, vorzugsweise 25 bis 60 Mol-%, insbesondere 30 bis 55 Mol-%, an anionischen Monomeren (a) im Verhältnis zur gesamten molaren Menge von Monomeren hergestellt wird.

3. Copolymer nach einem der Ansprüche 1 bis 2,
• bei dem das nicht-ionische hydrophobe Monomer (b) einem nicht-ionischen hydrophoben Monomer mit einer polymerisierbaren funktionellen Vinylgruppe entspricht, ausgewählt aus Methylacrylat, Ethylenacrylat, Butylacrylat, 2-Ethylhexylacrylat, Methylmethacrylat, Ethylmethacrylat, Butylmethacrylat und Mischungen davon; oder
• das durch Polymerisationsreaktion mit Umsetzung von 30 bis 80 Mol-%, vorzugsweise 35 bis 75 Mol-%, noch mehr bevorzugt 45 bis 70 Mol-%, an nicht-ionischen hydrophoben Monomeren (b) im Verhältnis zur gesamten molaren Menge von Monomeren hergestellt wird.

4. Copolymer nach einem der Ansprüche 1 bis 3,
• wobei das Monomer (c) ausgewählt ist aus einer Verbindung (c1) mit der Formel (I), wobei R¹ für H steht; R² für -C(H)=CH₂ steht; L¹ für CH₂-CH₂ und n für 10 steht; einer Verbindung (c2) mit der Formel (I), wobei R¹ für H steht; R² für -C(CH3)=CH2 steht_{;} L¹ für CH2-CH2 und n für 3,5 steht; einer Verbindung (c3) mit der Formel (I), wobei R¹ für H steht; L¹ für CH₂-CH₂ steht_{;} R² für -C(CH₃)=CH₂ und n für 10 steht; oder
• das durch Polymerisationsreaktion mit Umsetzung von weniger als 5 Mol-%, vorzugsweise 0,01 bis 5 Mol-% und noch mehr bevorzugt 0,02 bis 4 Mol-% oder 0,02 bis 2 Mol-% bzw. 0,04 bis 0,5 Mol-%, 0,04 bis 0,25 Mol-%, an Monomeren (c) im Verhältnis zur gesamten molaren Menge von Monomeren hergestellt wird.

5. Copolymer nach einem der Ansprüche 1 bis 4, das durch Polymerisationsreaktion ebenfalls mit Umsetzung mindestens eines nicht-ionischen Monomers (d), das sich vom Monomer (b) unterscheidet, bestehend aus einer polymerisierbaren funktionellen Vinylgruppe und einer Kohlenwasserstoffkette mit mindestens 10 Kohlenstoffatomen, hergestellt wird, vorzugsweise ausgewählt aus:
• einem Monomer (d1) mit einer polymerisierbaren funktionellen Vinylgruppe und einer C₁₂-C₃₆-Kohlenwasserstoffkette;
• einem Monomer (d2) mit einer polymerisierbaren funktionellen Vinylgruppe, einer C₁₂-C₃₆-Kohlenwasserstoffkette und 1 bis 150, vorzugsweise 15 bis 50 und noch mehr bevorzugt 20 bis 30, Alkylenoxygruppen, vorzugsweise einem Monomer (d2) mit der Formel (II):
T¹-(CH₂CH₂O)ₘ(CH₂CH(CH₃)O)ₚ-(CH₂CH(CH₂CH₃)O)_{q}-T² (II)
wobei:
o T¹ unabhängig eine polymerisierbare funktionelle Vinylgruppe darstellt;
o T² unabhängig Folgendes darstellt:
▪ eine Kohlenwasserstoffkette mit mindestens 10 Kohlenstoffatomen, vorzugsweise eine C₁₂-C₃₆-Kohlenwasserstoffkette; oder
▪ eine Kohlenwasserstoffkette mit mindestens 10 Kohlenstoffatomen, vorzugsweise eine C₁₂-C₃₆-Kohlenwasserstoffkette, und mindestens ein Heteroatom ausgewählt aus O, S, N oder P; oder
o m, p und q, die gleich oder verschieden sein können, unabhängig eine Ganz- oder Dezimalzahl von 0 bis 150 darstellen, wobei die Summe aus m, p und q ungleich null ist;
das vorzugsweise durch Polymerisationsreaktion mit Umsetzung von 0,01 bis 10 Mol-% oder 0,02 bis 5 Mol-% oder 0,02 bis 2 Mol-% an Monomeren (d) im Verhältnis zur gesamten molaren Menge von Monomeren hergestellt wird.

6. Copolymer nach einem der Ansprüche 1 bis 5, das durch Polymerisationsreaktion ebenfalls mit Umsetzung mindestens eines ionischen oder nicht-ionischen Monomers (e), das sich vom Monomer (b) unterscheidet, hergestellt wird, ausgewählt aus:
- 2-Acrylamido-2-methylpropansulfonsäure oder eines ihrer Salze;
- Acrylsäuretelomeren, vorzugsweise Dimeren, Trimeren oder Tetrameren, die ungesättigt sind;
- Monomeren laut der Formel (III): 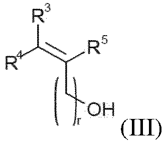 wobei:
▪ R³, R⁴ und R⁵ gleich oder verschieden sein können und unabhängig H oder CH₃ darstellen;
▪ r unabhängig 1, 2 oder 3 darstellt;
- Monomeren laut der Formel (IV), insbesondere HEMA, HPMA und HPA: 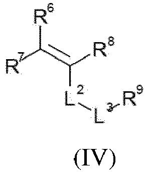 wobei:
▪ R⁶, R⁷ und R⁸ gleich oder verschieden sein können und unabhängig H oder CH3 darstellen;
▪ R⁹ für H oder CH₃ steht;
▪ L² unabhängig eine direkte Bindung oder eine Gruppe darstellt, ausgewählt aus O, C(O)O, CH₂CH₂ und CH₂;
▪ L³ unabhängig eine direkte Bindung oder 1 bis 150, vorzugsweise 15 bis 50 und noch mehr bevorzugt 25 bis 30, Alkylenoxygruppen darstellt;
das vorzugsweise durch Polymerisationsreaktion mit Umsetzung von 0,01 bis 25 Mol-% oder 0,02 bis 15 Mol-% oder 0,02 bis 10 Mol-% an Monomeren (e) im Verhältnis zur gesamten molaren Menge von Monomeren hergestellt wird.

7. Copolymer nach einem der Ansprüche 1 bis 6, das durch Polymerisationsreaktion ebenfalls mit Umsetzung mindestens eines anderen Monomers (f) herstellt wird, bevorzugt ausgewählt aus einer Verbindung laut der Formel (V), Trimethylolpropantrimethacrylat, Trimethylolpropantriacrylat, ethoxyliertem Trimethylolpropantrimethacrylat, ethoxyliertem Trimethylolpropantriacrylat, Ethylenglykoldimethacrylat, Ethylenglykoldiacrylat, Methylenbisacrylamid, Diallylphthalat, Diallylmaleat und Mischungen davon: 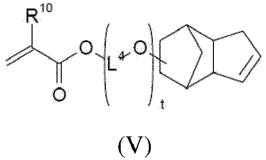 wobei:
• R¹⁰ unabhängig H oder CH3 darstellt;
• L⁴ unabhängig eine lineare oder verzweigte C₁-C₂₀-Alkylengruppe darstellt;
• t unabhängig 0 oder eine Ganzzahl von 1 bis 30, beispielsweise von 1 bis 20, insbesondere von 1 bis 15, besonders von 1 bis 10, darstellt;
das vorzugsweise durch Polymerisationsreaktion mit Umsetzung von weniger als 5 Mol-%, vorzugsweise 0,01 bis 4 Mol-%, insbesondere 0,02 bis 2 Mol-%, vor allem 0,04 bis 1 Mol-%, an Monomeren (f) im Verhältnis zur gesamten molaren Menge von Monomeren hergestellt wird.

8. Copolymer nach einem der Ansprüche 1 bis 7, das durch Polymerisationsreaktion ebenfalls mit Umsetzung mindestens eines anderen Monomers (g) gemäß Formel hergestellt wird:
• Verbindung laut der Formel (VI): 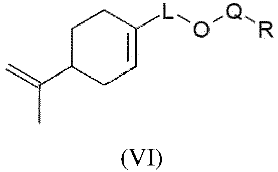 wobei:
- L für CH2, monoalkoxyliertes CH2 oder polyalkoxyliertes CH2 steht;
- Q eine direkte Bindung oder C(O) darstellt;
- R für -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ oder Q³OQ⁴OC(O)C(H)=CH₂steht;
- Q³ einen zweiwertigen Rest einer asymmetrischen Diisocyanatverbindung darstellt, vorzugsweise ausgewählt aus Tolyl-1,3-diisocyanat (TDI) und Isophorondiisocyanat (IPDI);
- Q⁴ für CH2, CH2-CH2, monoalkoxyliertes CH2, monoalkoxyliertes CH2-CH2, polyalkoxyliertes CH2 oder polyalkoxyliertes CH2-CH2 steht;
• Verbindung laut der Formel (VI-A): 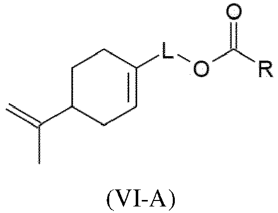 wobei:
- L für CH2, monoalkoxyliertes CH2 oder polyalkoxyliertes CH2 steht;
- R für -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ oder Q³OQ⁴OC(O)C(H)=CH₂steht;
- Q³ einen zweiwertigen Rest einer asymmetrischen Diisocyanatverbindung darstellt, vorzugsweise ausgewählt aus Tolyl-1,3-diisocyanat (TDI) und Isophorondiisocyanat (IPDI);
- Q⁴ für CH2, CH2-CH2, monoalkoxyliertes CH2, monoalkoxyliertes CH2-CH2, polyalkoxyliertes CH2 oder polyalkoxyliertes CH2-CH2 steht;
• Verbindung laut der Formel (VI-B): 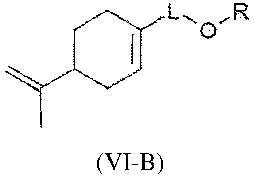 wobei:
- L für CH2, monoalkoxyliertes CH2 oder polyalkoxyliertes CH2 steht;
- R für -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH, Q³OQ⁴OC(O)C(CH₃)=CH₂ oder Q³OQ⁴OC(O)C(H)=CH₂steht;
- Q³ einen zweiwertigen Rest einer asymmetrischen Diisocyanatverbindung darstellt, vorzugsweise ausgewählt aus Tolyl-1,3-diisocyanat (TDI) und Isophorondiisocyanat (IPDI);
- Q⁴ für CH2, CH2-CH2, monoalkoxyliertes CH2, monoalkoxyliertes CH2-CH2, polyalkoxyliertes CH2 oder polyalkoxyliertes CH2-CH2 steht;
das vorzugsweise durch Polymerisationsreaktion mit Umsetzung von weniger als 5 Mol-%, vorzugsweise 0,01 bis 4 Mol-%, insbesondere 0,02 bis 4 Mol-% oder 0,02 bis 2 Mol-%, vor allem 0,04 bis 1 Mol-%, an Monomeren (g) im Verhältnis zur gesamten molaren Menge von Monomeren hergestellt wird.

9. Verwendung, für die Herstellung eines Copolymers (P1) nach einem der vorstehenden Ansprüche, mit weniger als 5 Mol-%, bezogen auf die gesamte molare Menge von Monomeren, mindestens eines Monomers laut der Formel (I): 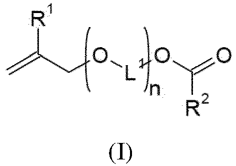 wobei:
• R¹ unabhängig H oder CH3 darstellt;
• R² unabhängig -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH darstellt;
• L¹ unabhängig eine Ethylen-, Propylen- oder Butylen-Gruppe darstellt;
• n unabhängig 0 oder eine Ganz- bzw. Dezimalzahl von 1 bis 30 darstellt.

10. Verfahren (1) zur Herstellung eines Copolymers (P1) durch Polymerisationsreaktion:
(a) mindestens eines anionischen Monomers mit mindestens einer polymerisierbaren ethylenischen Ungesättigtheit, vorzugsweise eines anionischen Monomers mit einer polymerisierbaren funktionellen Vinylgruppe und mindestens einer Carbonsäure-Funktion; und
(b) mindestens eines nicht-ionischen hydrophoben Monomers mit mindestens einer polymerisierbaren ethylenischen Ungesättigtheit, ausgewählt aus C₁-C₈-Alkylacrylaten, C₁-C₈-Alkylmethacrylaten, C₁-C₈-Alkylmaleaten, C₁-C₈-Alkylitaconaten, de C₁-C₈Alkylcrotonaten, C₁-C₈-Alkylcinnamaten und Mischungen davon; und
(c) mindestens eines Monomers mit der Formel (I): 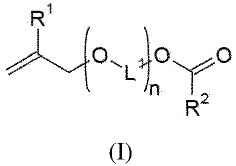 wobei:
• R¹ unabhängig H oder CH3 darstellt;
• R² unabhängig -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH darstellt;
• L¹ unabhängig eine Ethylen-, Propylen- oder Butylen-Gruppe darstellt;
• n unabhängig 0 oder eine Ganz- bzw. Dezimalzahl von 1 bis 30 darstellt.

11. Verfahren (2) zur Herstellung eines Copolymers (P2) durch Polymerisationsreaktion nach Anspruch 10 und ebenfalls mit Umsetzung eines Copolymers (P1) bei der Polymerisationsreaktion, das zuvor durch Polymerisationsreaktion nach Anspruch 1 bis 8 hergestellt wurde.

12. Copolymer (P2), das gemäß Verfahren (2) nach Anspruch 11 hergestellt werden kann, vorzugsweise ein mehrphasiges Copolymer (P2) oder ein Copolymer mit einem Polymerkern mit einem ersten Copolymer (P1), das vollständig oder teilweise von einem zweiten Copolymer (P1) überzogen ist, das mit dem ersten Copolymer (P1) identisch oder verschieden ist; wobei insbesondere das Gewichtsverhältnis 1. Copolymer (P1)/2. Copolymer (P1) zwischen 45/55 und 95/5, insbesondere zwischen 60/40 und 90/10, liegt.

13. Wässrige Zusammensetzung mit mindestens einem Copolymer (P1) nach einem der Ansprüche 1 bis 8 oder mindestens einem Copolymer (P2) nach Anspruch 12.

14. Kosmetische Zusammensetzung, insbesondere kosmetische Zusammensetzung mit einem pH-Wert von 3 bis 9, vorzugsweise von 3 bis 7, noch mehr bevorzugt von 4 bis 7, bestehend aus:
• mindestens einem Copolymer (P1) nach einem der Ansprüche 1 bis 8; oder
• mindestens einem Copolymer (P2) nach Anspruch 12; oder
• mindestens einem Copolymer (P1) nach einem der Ansprüche 1 bis 8 und mindestens einem Copolymer (P2) nach Anspruch 12; oder
• mindestens einer wässrigen Zusammensetzung nach Anspruch 13.

15. Verwendung, zur Herstellung einer wässrigen Zusammensetzung nach Anspruch 13 oder zur Herstellung einer kosmetischen Zusammensetzung nach Anspruch 14,
• mindestens eines Copolymers (P1) nach einem der Ansprüche 1 bis 8; oder
• mindestens eines Copolymers (P2) nach Anspruch 12; oder
• mindestens eines Copolymers (P1) nach einem der Ansprüche 1 bis 8 und mindestens eines Copolymers (P2) nach Anspruch 12.

## Claims

1. Copolymer (P1) obtained by reaction for polymerization:
(a) of at least one anionic monomer comprising at least one polymerizable ethylenic unsaturation; and
(b) of at least one hydrophobic nonionic monomer comprising at least one polymerizable ethylenic unsaturation and chosen from C₁-C₈-alkyl acrylates, C₁-C₈-alkyl methacrylates, C₁-C₈-alkyl maleates, C₁-C₈-alkyl itaconates, C₁-C₈-alkyl crotonates, C₁-C₈-alkyl cinnamates, and mixtures thereof; and
(c) of at least one monomer of formula (I): 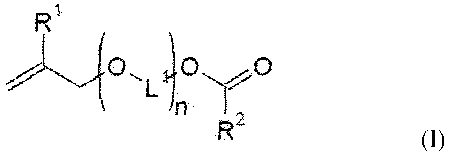 wherein:
• R1 independently represents H or CH3;
• R2 independently represents
-C(H)=CH2, -C(CH3)=CH2, -C(H)=C(H)C(O)OH, -C(H)=C(H)CH3, -C(=CH2)CH2C(O)OH, -CH2C(=CH2)C(O)OH;
• L1 independently represents an ethylene, propylene or butylene group;
• n independently represents 0 or an integer or decimal ranging from 1 to 30.

2. Copolymer according to Claim 1:
• for which the anionic monomer (a) is an anionic monomer comprising a polymerizable vinyl function and at least one carboxylic acid function, preferably a monomer chosen from acrylic acid, methacrylic acid, maleic acid, itaconic acid, crotonic acid, an acrylic acid salt, a methacrylic acid salt, a maleic acid salt, an itaconic acid salt, a crotonic acid salt, a cinammic acid salt; and mixtures thereof; or
• obtained by a polymerization reaction using at least 20 mol%, preferably from 25 to 60 mol%, in particular from 30 to 55 mol% of anionic monomer (a), relative to the total molar amount of monomers.

3. Copolymer according to either of Claims 1 and 2:
• for which the hydrophobic nonionic monomer (b) is a hydrophobic nonionic monomer comprising a polymerizable vinyl function, chosen from methyl acrylate, ethyl acrylate, butyl acrylate, 2-ethyl-hexyl acrylate, methyl methacrylate, ethyl methacrylate, butyl methacrylate, and mixtures thereof; or
• obtained by a polymerization reaction using from 30 to 80 mol%, preferably from 35 to 75 mol%, more preferentially from 45 to 70 mol% of hydrophobic nonionic monomer (b), relative to the total molar amount of monomers.

4. Copolymer according to one of Claims 1 to 3:
• for which the monomer (c) is chosen from a compound (c1) of formula (I) wherein R¹ represents H; R² represents -C(H)=CH₂; L¹ represents CH₂-CH₂ and n represents 10; a compound (c2) of formula (I) wherein R¹ represents H; R² represents -C(CH₃)=CH₂; L¹ represents CH₂-CH₂ and n represents 3.5; a compound (c3) of formula (I) wherein R¹ represents H; L¹ represents CH2-CH2; R² represents -C(CH₃)=CH₂ and n represents 10; or
• obtained by a polymerization reaction using less than 5 mol%, preferably from 0.01 to 5 mol%, and more preferentially from 0.02 to 4 mol%, or from 0.02 to 2 mol% or from 0.04 to 0.5 mol% or from 0.04 to 0.25 mol% of monomer (c), relative to the total molar amount of monomers.

5. Copolymer according to one of Claims 1 to 4, obtained by a polymerization reaction also using at least one nonionic monomer (d), different than the monomer (b), comprising a polymerizable vinyl function and a hydrocarbon-based chain comprising at least 10 carbon atoms, preferably chosen from:
• a monomer (d1) comprising a polymerizable vinyl function and a C₁₂-C₃₆ hydrocarbon-based chain;
• a monomer (d2) comprising a polymerizable vinyl function, a C₁₂-C₃₆ hydrocarbon-based chain and from 1 to 150, preferably from 15 to 50 and more preferentially from 20 to 30 alkylene-oxy groups, preferably a monomer (d2) of formula (II):
T¹-(CH₂CH₂O)ₘ-(CH₂CH(CH₃)O)ₚ-(CH₂CH(CH₂CH₃)O)_{q}-T² (II)
wherein:
o T¹ independently represents a polymerizable vinyl function;
o T² independently represents:
▪ a hydrocarbon-based chain comprising at least 10 carbon atoms, preferably a C₁₂-C₃₆ hydrocarbon-based chain; or
▪ a hydrocarbon-based chain comprising at least 10 carbon atoms, preferably a C₁₂-C₃₆ hydrocarbon-based chain, and at least one heteroatom chosen from O, S, N and P; and
▪ m, p and q, which may be identical or different, independently represent an integer or decimal ranging from 0 to 150, the sum of m, p and q being non-zero;
preferably obtained by a polymerization reaction using from 0.01 to 10 mol%, or from 0.02 to 5 mol% or from 0.02 to 2 mol% of monomer (d), relative to the total molar amount of monomers.

6. Copolymer according to one of Claims 1 to 5, obtained by a polymerization reaction also using at least one ionic or nonionic monomer (e), different than the monomer (b), chosen from:
- 2-acrylamido-2-methylpropane sulfonic acid or a salt thereof;
- telomers, preferably dimers, trimers or tetramers, which are unsaturated, of acrylic acid;
- the monomers of formula (III): 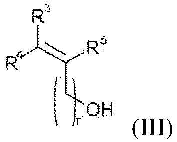 wherein:
▪ R³, R⁴ and R⁵, which may be identical or different, independently represent H or CH₃ and
▪ r independently represents 1, 2 or 3;
- the monomers of formula (IV), in particular HEMA, HPMA and HPA: 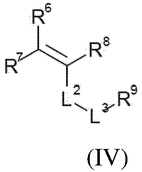 wherein:
▪ R⁶, R⁷ and R⁸, which may be identical or different, independently represent H or CH₃;
▪ R⁹ represents H or CH₃;
▪ L² independently represents a direct bond or a group chosen from O, C(O)O, CH₂CH₂ and CH₂;
▪ L³ independently represents a direct bond or from 1 to 150, preferably from 15 to 50 and more preferentially from 25 to 30 alkylene-oxy groups;
preferably obtained by a polymerization reaction using from 0.01 to 25 mol%, or from 0.02 to 15 mol% or from 0.02 to 10 mol% of monomer (e), relative to the total molar amount of monomers.

7. Copolymer according to one of Claims 1 to 6, obtained by a polymerization reaction also using at least one other monomer (f), preferably chosen from a compound of formula (V), trimethylolpropane trimethacrylate, trimethylolpropane triacrylate, ethoxylated trimethylolpropane trimethacrylate, ethoxylated trimethylolpropane triacrylate, ethylene glycol dimethacrylate, ethylene glycol diacrylate, methylenebisacrylamide, diallyl phthalate, diallyl maleate, and mixtures thereof: 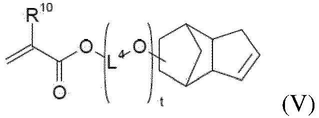 wherein:
• R¹⁰ independently represents H or CH₃;
• L⁴ independently represents a linear or branched C₁-C₂₀-alkylene group;
• t independently represents 0 or an integer ranging from 1 to 30, for example from 1 to 20, in particular from 1 to 15, in particular from 1 to 10;
preferably obtained by a polymerization reaction using less than 5 mol%, preferably from 0.01 to 4 mol%, in particular from 0.02 to 2 mol%, in particular from 0.04 to 1 mol%, of monomer (f), relative to the total molar amount of monomers.

8. Copolymer according to one of Claims 1 to 7, obtained by a polymerization reaction also using at least one other monomer (g) of formula
• a compound of formula of formula (VI): 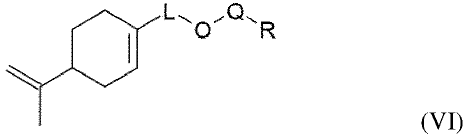 wherein:
- L represents CH2, monoalkoxylated CH2 or polyalkoxylated CH2;
- Q represents a direct or C(O) bond,
- R represents -C(H)=CH₂, -C(CH₃)=CH₂, -C(H)=C(H)C(O)OH, - C(H)=C(H)CH₃, -C(=CH₂)CH₂C(O)OH, -CH₂C(=CH₂)C(O)OH, Q³OQ⁴OC(O)C(CH₃)=CH₂ or Q³OQ⁴OC(O)C(H)=CH₂;
- Q³ represents a divalent residue of an asymmetric diisocyanate compound, preferably chosen from tolyl-1,3-diisocyanate (TDI) and isophorone-diisocyanate (IPDI);
- Q⁴ represents CH2, CH2-CH2, monoalkoxylated CH2, monoalkoxylated CH2-CH2, polyalkoxylated CH2 or polyalkoxylated CH2-CH2;
• a compound of formula (VI-A): 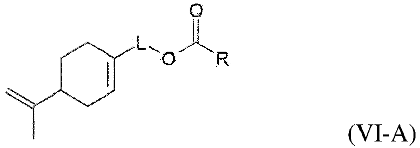 wherein:
- L represents CH2, monoalkoxylated CH2 or polyalkoxylated CH2;
- R represents -C(H)=CH₂, -C(CH₃)=CH₂, -C(H)=C(H)C(O)OH, - C(H)=C(H)CH₃, -C(=CH₂)CH₂C(O)OH, -CH₂C(=CH₂)C(O)OH, Q³OQ⁴OC(O)C(CH₃)=CH₂ or Q³OQ⁴OC(O)C(H)=CH₂;
- Q³ represents a divalent residue of an asymmetric diisocyanate compound, preferably chosen from tolyl-1,3-diisocyanate (TDI) and isophorone-diisocyanate (IPDI);
- Q⁴ represents CH2, CH2-CH2, monoalkoxylated CH2, monoalkoxylated CH2-CH2, polyalkoxylated CH2 or polyalkoxylated CH2-CH2;
• a compound of formula (VI-B): 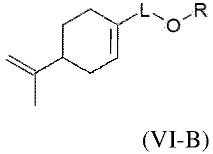 wherein:
- L represents CH2, monoalkoxylated CH2 or polyalkoxylated CH2;
- R represents -C(H)=CH₂, -C(CH₃)=CH₂, -C(H)=C(H)C(O)OH, - C(H)=C(H)CH₃, -C(=CH₂)CH₂C(O)OH, -CH₂C(=CH₂)C(O)OH, Q³OQ⁴OC(O)C(CH₃)=CH₂ or Q³OQ⁴OC(O)C(H)=CH₂;
- Q³ represents a divalent residue of an asymmetric diisocyanate compound, preferably chosen from tolyl-1,3-diisocyanate (TDI) and isophorone-diisocyanate (IPDI);
- Q⁴ represents CH2, CH2-CH2, monoalkoxylated CH2, monoalkoxylated CH2-CH2, polyalkoxylated CH2 or polyalkoxylated CH2-CH2;
preferably obtained by a polymerization reaction using less than 5 mol %, preferably from 0.01 to 4 mol %, in particular from 0.02 to 4 mol % or from 0.02 to 2 mol %, in particular from 0.04 to 1 mol % of monomer (g), relative to the total motor amount of monomers.

9. Use, for the preparation of a copolymer (P1) according to any of the previous claims, of less than 5 mol%, relative to the total molar amount of monomers, of at least one monomer of formula (I): 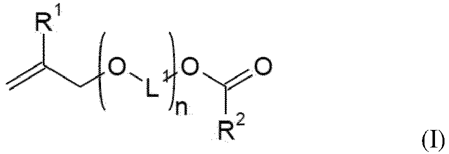 wherein:
• R1 independently represents H or CH3;
• R2 independently represents
-C(H)=CH2, -C(CH3)=CH2, -C(H)=C(H)C(O)OH, -C(H)=C(H)CH3, -C(=CH2)CH2C(O)OH, -CH2C(=CH2)C(O)OH;
• L1 independently represents an ethylene, propylene or butylene group;
• n independently represents 0 or an integer or decimal ranging from 1 to 30.

10. Process (1) for producing a copolymer (P1) obtained by a reaction for polymerization:
(a) of at least one anionic monomer comprising at least one polymerizable ethylenic unsaturation, preferably an anionic monomer comprising a polymerizable vinyl function and at least one carboxylic acid function; and
(b) of at least one hydrophobic nonionic monomer comprising at least one polymerizable ethylenic unsaturation, chosen from C₁-C₈-alkyl acrylates, C₁-C₈-alkyl methacrylates, C₁-C₈-alkyl maleates, C₁-C₈-alkyl itaconates, C₁-C₈-alkyl crotonates, C₁-C₈-alkyl cinnamates, and mixtures thereof, and
(c) of at least one monomer of formula (I): 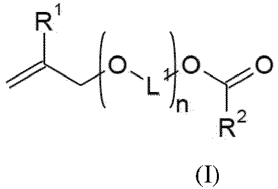 wherein:
• R1 independently represents H or CH3;
• R2 independently represents
-C(H)=CH2, -C(CH3)=CH2, -C(H)=C(H)C(O)OH, -C(H)=C(H)CH3, -C(=CH2)CH2C(O)OH, -CH2C(=CH2)C(O)OH;
• L1 independently represents an ethylene, propylene or butylene group;
• n independently represents 0 or an integer or decimal ranging from 1 to 30.

11. Process (2) for producing a copolymer (P2) obtained by a polymerization reaction as defined in Claim 10 and also comprising the use, during the polymerization reaction, of a copolymer (PI), obtained beforehand by a polymerization reaction according to one of Claims 1 to 8.

12. Copolymer (P2) which can be obtained according to the process (2) of Claim 11, preferably a copolymer (P2) which is multiphase or else which comprises a core comprising a first copolymer (PI), totally or partially covered with a second copolymer (PI), which may be identical to or different than the first copolymer (P1) ; in particular for which the 1° copolymer (P1)/2° copolymer (P1) weight ratio is between 45/55 and 95/5, in particular between 60/40 and 90/10.

13. Aqueous composition comprising at least one copolymer (P1) according to one of Claims 1 to 8 or at least one copolymer (P2) according to Claim 12.

14. Cosmetic composition, in particular cosmetic composition for which the pH ranges from 3 to 9, preferably from 3 to 7, more preferentially from 4 to 7, comprising:
• at least one copolymer (P1) according to one of Claims 1 to 8; or
• at least one copolymer (P2) according to Claim 12; or
• at least one copolymer (P1) according to one of Claims 1 to 8 and at least one copolymer (P2) according to Claim 12; or
• at least one aqueous composition defined according to Claim 13.

15. Use, for the production of an aqueous composition according to Claim 13 or for the production of a cosmetic composition according to Claim 14,
• of at least one copolymer (P1) according to one of Claims 1 to 8; or
• of at least one copolymer (P2) according to Claim 12; or
• of at least one copolymer (P1) according to one of Claims 1 to 8 and of at least one copolymer (P2) according to Claim 12.
